(19) **Europäisches Patentamt European Patent Office Office européen des brevets**

(11) **EP 2 115 108 B1**

(12) **EUROPEAN PATENT SPECIFICATION**

(45) Date of publication and mention of the grant of the patent:
**24.07.2013 Bulletin 2013/30**

(21) Application number: **08729500.2**

(22) Date of filing: **11.02.2008**

(51) Int Cl.:
*C11D 1/00* (2006.01)   *C11D 3/00* (2006.01)

(86) International application number:
**PCT/US2008/053547**

(87) International publication number:
**WO 2008/100842 (21.08.2008 Gazette 2008/34)**

(54) **COMPOSITE**

VERBUNDSTOFFE

COMPOSITE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **12.02.2007 US 900968 P**

(43) Date of publication of application:
**11.11.2009 Bulletin 2009/46**

(73) Proprietor: **Dow Global Technologies LLC Midland, MI 48674 (US)**

(72) Inventors:
• **STOCKTON, Luther Midland, MI 48642 (US)**
• **MACHELSKI, Susan, Marie Midland, MI 48640 (US)**
• **STRAND, Deidre, Ann Midland, MI 48642 (US)**
• **FOWLER, Harold, Christian Midland, MI 48640 (US)**

• **KIM, Young-Sam Midland, Texas 48640 (US)**
• **CLAASEN, Gert, Johannes CH-8805 Richterswil (CH)**
• **STOLLMAIER, Friederike, T. D-77836 Rheinmuenster (DE)**
• **THIEDE, Verena, M. T. D-48317 Muenster (DE)**
• **ZHANG, Xiaodong Livingston, NJ 07039 (US)**
• **KATZER, Karin CH-8810 Horgen (CH)**

(74) Representative: **Hayes, Adrian Chetwynd Boult Wade Tennant Verulam Gardens 70 Gray's Inn Road London WC1X 8BT (GB)**

(56) References cited:
**WO-A-2004/046214     WO-A-2004/078900
WO-A-2005/019241     WO-A-2006/060520
WO-A-2007/100312**

**Description**

BACKGROUND OF INVENTION

Field of the Invention

Embodiments disclosed herein relate generally to polymeric composite structures. In some embodiments, the composite structure may include a substrate having a desired combination of performance properties including high softness and high loft. More specifically, embodiments disclosed herein relate to polymeric composite structures that have an open-cell foam layer, a substrate layer, and optionally at least one cleaning surfactant, active agent, or enhancing filler.

Background

Dry and wet, or pre-moistened, wipes are well known consumer products available in many forms. Dry wipes may include a substrate, with or without additives, such as antibacterial substances or cleansing agents that may be released upon contact with skin, oil, or water. Wet wipes include a substrate, such as a non-woven web, which may be pre-moistened with a mild surfactant-based solution, and may include lotions, cleansing agents, or other additives. Such wet and dry wipes have been used for baby wipes, hand wipes, household cleaning wipes, industrial wipes, body and facial wipes, and the like. Typically, wipes are provided as either folded, stacked sheets or as a perforated roll, where the sheets are meant to be used one at a time.

Initially, wet wipe products were made of traditional non-woven materials based on paper making technology (pulp based products). These products were well accepted but deficient in softness of the fabric material. The introduction of spunlace non-woven technology offered products that, compared to traditional paper based products, were superior in terms of softness. This is mainly due to (i) the use of long soft fibers (most frequently rayon and polyethylene terephthalate/polypropylene or a mixture of these fibers) in the spunlace process and (ii) the fact that during the spunlace process no binder is added to the fabric.

Other conventional wet wipes have included a single layer of a substantially homogeneous material. For example, conventional wet wipes have included an air laid web of fibers that are uniformly mixed or distributed throughout the web. The wipes have included polymeric fibers, such as polyester, polyethylene, and polypropylene, and natural or synthetic fibers, such as cellulosic fibers. Other conventional wet wipes have included a co-formed web of polypropylene and cellulosic fibers uniformly mixed throughout the web.

However, other forms of a wet wipe or wipe-type product include a wipe product having a non-woven, layered base sheet. The layered base sheet may include at least two layers positioned in facing relation with each other where one of the layers includes fibers that are not included in the other layer, such as where one layer includes polyethylene fibers and one layer includes polypropylene fibers. In alternate forms, the layers may include similar materials, but in differing amounts. One layer may be configured to provide different physical properties, such as softness, to the wipe product while another layer may be configured to provide other properties, such as strength, to the wipe product. WO 1998/003713, corresponding to US Patent No. 6,028,018 discloses one example of a wet wipe having a multilayer base sheet.

WO-A-2007/100312 describes a composition which contains a mixture of a shear thickening fluid and at least one inert filler and said shear thickening fluid and filler remain in a conformable form.

WO-A-2006/060520 relates to processes and apparati for selectively electroplating a metal layer or layers into recessed topographic features on a conductive surface.

WO-A-20041046214 relates to a polymer comprising one or more $C_3$ to $C_{40}$ olefins, optionally one or more diolefins, and less than 15 mole % of ethylene, where the polymer has a) a Dot-T-Peel of 1 Newton or more; and b) a branching index (g') of 0.95 or less measured at the $M_z$ of the polymer; c) an $M_w$ of 100,000 or less.

WO-A-2005/019241 relates to compositions of novel antimicrobial peptides. The peptides exhibit high antibacterial activity and low hemolytic activity.

WO-A-2004/078900 discloses foamed polyurethane articles comprising one or more quaternary ammonium compounds having germicidal properties.

A recent innovation to improve loft of wipes includes the controlled formation of machine direction voids within a spunlaced non-woven. Void formation may be introduced to a spunlaced non-woven by placing stationary parallel tubes between two case of polyolefins has a residence time of about 0.5 to 10 hours in the purge column. A flow of nitrogen and of residual gas exits the purge column via a gas outlet and is directed to an appropriate treatment device via a gas discharge line.

DE 41 27 810 A1 discloses degassing a solution by evacuating and airing it with an inert gas several times.

DD 152 729 A1 discloses degassing rocks by flushing an inert gas in an evacuated gastight mill.

DE 24 49 787 A1 discloses degassing polymer melt and polymer solutions in an extruder.

The inventors have found out that in a process of polymerising ethylene, the amount of undesired hydrocarbon gases in the Final polyethylene powder (i.e. before pelletising) is too high for some applications, such as for applications where the final product is in contact with food.

OBJECTS AND SUMMARY OF THE INVENTION

It is an object of the present invention to provide a method for degassing a polymer powder that is more effective than the currently known methods. It is also an object to further decrease the amount of hydrocarbons at a powder outlet especially in a manufacturing process for polyethylene.

A further object of the present invention is to minimise the losses of residual gases and to decrease the costs of production. A yet another object of the invention is to enhance the recycling of the gases used in a polymer manufacturing process, such as the flushing gas.

At least one of the above objects is at least partially achieved by the means of the present invention, namely a method for degassing polymer powder comprising flushing the polymer powder in a first chamber with a first flow of flushing gas, transferring the polymer powder into a second chamber and flushing the polymer powder with a second flow of flushing gas in said second chamber.

The present invention further relates to a system for degassing polymer powder comprising a first chamber equipped with at least one gas inlet and at least one gas outlet for a flushing gas; means for transferring the polymer powder from said first chamber into a second chamber, and a second chamber equipped with at least one gas inlet and at least one gas outlet for a flushing gas.

be used for wet wipes are stiffer and can provide strength and resiliency, but are not as soft or flexible as other fibers. Other fibers that may be used for wet wipes are softer but may not have sufficient wet strength to withstand the forces exerted by the user. Moreover, the different types of fibers that may provide the desired properties, such as fibers for strength and fibers for softness, have been difficult to combine in a homogenous layer due to incompatibilities with each other.

Accordingly, there exists a need for wet wipes with improved softness and flexibility while maintaining the strength, integrity, resiliency, fuzz resistance, and other properties of the wipes.

SUMMARY OF THE INVENTION

The present invention relates to a composite structure comprising:

at least one substrate layer;
at least one layer comprising an open-cell polyolefin foam having a density in the range of from 0.03 to 0.07 g/cc disposed on the substrate layer;
wherein the substrate layer comprises a polyurethane or polyolefin non-woven having a basis weight of 15 to 250 grams per square meter; and
wherein the composite structure has a basis weight of 15 to 500 grams per square meter.

A method of forming a composite structure including at least one substrate layer and at least one layer comprising an open-cell foam is also described. The method may include applying a froth to a substrate, wherein the froth comprises water and a thermoplastic polymer, and removing at least a portion of the water from the froth to form a foam.

Other aspects and advantages of the invention will be apparent from the following description and the appended claims.

BRIEF DESCRIPTION OF THE DRAWINGS

Figure 1 illustrates the formation of foam from froth in accordance with embodiments disclosed herein.
Figure 2 schematically illustrates an extrusion apparatus that may be used in embodiments disclosed herein.
Figure 3 is a micrograph of a cross-section of one embodiment of the composite structures disclosed herein
Figure 4 is a graphical representation of bending rigidity test results for embodiments of the composite structures disclosed herein as compared to commercially available comparative samples.
Figure 5 is a graphical representation of the fuzz resistance test results for embodiments of the composite structures disclosed herein as compared to commercially available comparative samples.
Figure 6 is a graphical representation of the results for hand measurements normalized by sample basis weight for embodiments of the composite structures disclosed herein as compared to commercially available comparative samples

Figure 7 is a graphical representation of the results for hand measurements normalized by sample volume for embodiments of the composite structures disclosed herein as compared to commercially available comparative samples

Figure 8 is a graphical representation of the results for Kawabata Evaluation System measurements for compression resilience for embodiments of the composite structures disclosed herein as compared to commercially available comparative samples

Figure 9-11 are graphical representations of the PPT tear strength test results for embodiments of the composite structures disclosed herein as compared to commercially available comparative samples.

Figure 12 is a graphical representation of the results for Kawabata Evaluation System measurements for geometric roughness for embodiments of the composite structures disclosed herein as compared to commercially available comparative samples.

Figure 13 is a graphical representation of the results for Kawabata Evaluation System measurements for coefficient of friction for embodiments of the composite structures disclosed herein as compared to commercially available comparative samples.

DETAILED DESCRIPTION

In one aspect, embodiments disclosed herein relate to composite structures having a balance of softness, weight, and other properties which may include bending rigidity, coefficient of friction, fuzz resistance, loft, volume, and others.

In other aspects, embodiments disclosed herein relate to composite structures having a balance of properties, wherein the composite structure includes at least one substrate layer and at least one open-cell foam layer. The substrate may include non-wovens, fabrics, and the like. Incorporation of open-cell foams with a substrate (e.g., non-wovens, fabrics, etc.) into wipes or other articles may impart additional softness, loft, and volume to the article. The additional loft and volume may be achieved while enhancing and/or maintaining the desired, pre-existing surface feel of the substrate alone. The incorporation of the open-cell foam may also increase the available void volume and/or surface area for the inclusion and delivery of active agents when compared to the fabric or non-woven layer alone.

In other aspects, embodiments disclosed herein relate to a composite structure including an open-cell foam layer, a substrate layer, and optionally at least one cleaning surfactant, active agent, or enhancing filler. Embodiments of the composite structure may exhibit a desired combination of performance properties, including high softness and high loft, and/or excellent resistance to surface abrasion. The soft, high loft composite structure may be useful for disposable and semi-disposable applications related to personal care, medical, shipping, and household markets. The composite structure may also be capable of delivering wet active agents or dry active agents requiring wetting for cleansing, polishing, or medical applications.

The composite structures disclosed herein may be used for cleaning wipes for skin contact, and may include wet and/or dry active agents. The composite structures disclosed herein may also be used for other applications including baby wipes, hand wipes, hard surface cleaners for home use, and industrial cleaning wipes.

Enhanced softness or a more cloth-like feel are also desirable for applications beyond skin cleansing wipes. These applications may include, but are not limited to, applicator pads, polishing cloths, medical cleansing, shipping/ packaging material for sensitive components, or application pads for topical medicines. Additionally these articles may be used as a means for the temporary storage of measured amounts liquid materials

Foams useful in embodiments of the composite structures disclosed herein may be formed from froths or frothed dispersions. As used herein, the terms "frothing" or "frothed" refers to a process where substantial volumes of air, or other gas, are incorporated in a liquid where, in some embodiments, at least 80 volume percent of the resulting composition (the frothed material) consists of the gaseous component. In other embodiments, at least 85 volume percent of the frothed material consists of the gaseous component; and at least 90 volume percent in yet other embodiments. The liquid may be a molecular solution, a micellar solution, or a dispersion in an aqueous or organic medium. In general the frothed liquid is created by mechanical methods such as high shear mixing under atmospheric conditions or optionally injecting gas into the system while mixing. The term "froth," as used herein, refers to a liquid which has been frothed, as described above, before drying or removing the liquid medium.

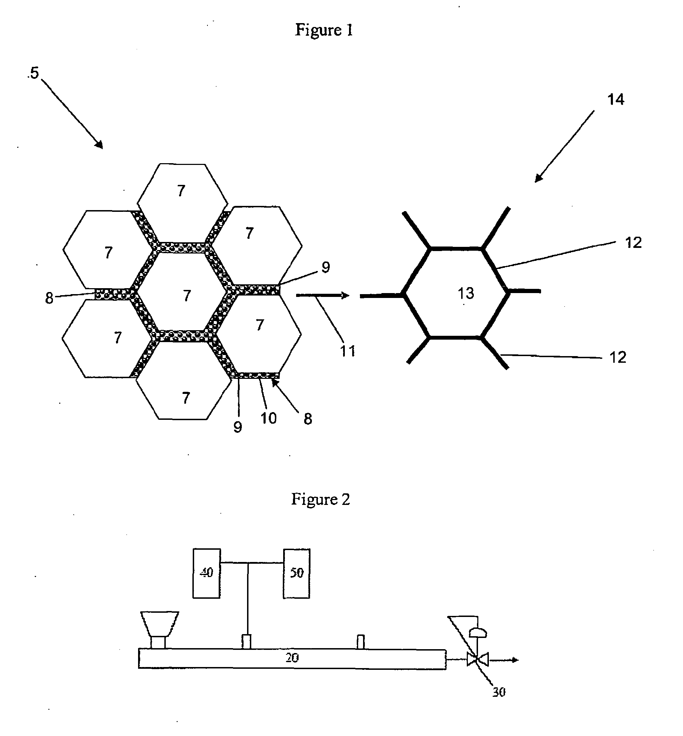

The term "foam," as used herein, refers to a resilient structure formed by removing a portion of the liquid medium from a froth, i.e., at least a portion, a substantial portion, or all of the liquid medium may be removed. As used herein, drying and removing may be used interchangeably, and may include thermal and/or mechanical removal of the liquid medium. The formation of a foam from a froth in accordance with embodiments disclosed herein is illustrated in Figure 1. A froth 5 may include pockets of vapor 7 within dispersion 8, where the dispersion 8 includes polymer particles 10 in a liquid medium 9. When the liquid medium 9 is removed from the froth 5 during a drying or removing process 11, the polymer particles 10 coalesce and melt together creating interconnected film or struts 12 around the entrapped vapor bubbles 13, giving stability to the resulting structure 14. Film formation may depend upon variables including the melting point of polymers within the froth, the rate of removal (*i.e.*, evaporation rate) of the liquid medium, and overall froth composition, among others. For example, as water is removed from a froth formed from an aqueous dispersion, polymers contained in the dispersion may coalesce, forming a film, giving structure and resiliency to the resulting foam. In some embodiments, a foam may be formed where the amount of residual liquid ranges from 0 to 20 weight percent; 0 to 10 weight percent in other embodiments; and 0 to 8 percent in yet other embodiments.

As described above, embodiments of the present disclosure include various substrates, including non-wovens, fabrics, and foams. Additionally, embodiments disclosed herein may include various additives, including wet or dry active agents. Each of these components and methods to form the composite structures disclosed herein are described in more detail below.

**FOAMS AND FOAM SUBSTRATES**

Foams useful in embodiments include foams formed from polyolefin resins

In some embodiments, polyolefin foams may be made from aqueous dispersions. The aqueous dispersions may be frothed and at least partially dried to result in the desired foams. Dispersions used in embodiments of the present disclosure may include water, at least one thermoplastic resin, and a dispersion stabilizing agent. The thermoplastic resin included in embodiments of the foams of the present disclosure may include a resin that is not readily dispersible in water by itself. The term "resin," as used herein, should be construed to include synthetic polymers or chemically modified natural resins. The thermoplastic resin includes polyolefins. Dispersions may also include various additives, including frothing surfactants. Each of these is discussed in more detail below.

**Polyolefin Resin**

Polyolefin resins used herein may include olefin polymers and elastomers, and blends of various olefin polymers and/or olefin elastomers. In some embodiments, the olefin resin is a semicrystalline resin. The term "semi-crystalline" is intended to identify those resins that possess at least one endotherm when subjected to standard differential scanning calorimetry (DSC) evaluation. Some semi-crystalline polymers exhibit a DSC endotherm that exhibits a relatively gentle slope as the scanning temperature is increased past the final endotherm maximum. This reflects a polymer of broad melting range rather than a polymer having what is generally considered to be a sharp melting point. Some polymers useful in the dispersions of the disclosure have a single melting point while other polymers have more than one melting point.

In some polymers, one or more of the melting points may be sharp such that all or a portion of the polymer melts over a fairly narrow temperature range, such as a few degrees centigrade. In other embodiments, the polymer may exhibit broad melting characteristics over a range of 20°C. In yet other embodiments, the polymer may exhibit broad melting characteristics over a range of greater than 50°C.

Examples of the olefin resins that may be used in the present disclosure include homopolymers and copolymers (including elastomers) of an alpha-olefin such as ethylene, propylene, 1-butene, 3-methyl-1-butene, 4-methyl-1-pentene, 3-methyl-1- pentene, 1-heptene, 1-hexene, 1-octene, 1-decene, and 1-dodecene, as typically represented by polyethylene, polypropylene, poly-1-butene, poly-3-methyl-1-butene, poly-3-methyl-1-pentene, poly-4-methyl-1-pentene, ethylene-propylene copolymer, ethylene-1-butene copolymer, and propylene- 1-butene copolymer; copolymers (including elastomers) of an alpha-olefin with a conjugated or non-conjugated diene, as typically represented by ethylene-butadiene copolymer and ethylene-ethylidene norbornene copolymer; and polyolefins (including elastomers) such as copolymers of two or more alpha-olefins with a conjugated or non-conjugated diene, as typically represented by ethylene-propylene-butadiene copolymer, ethylene-propylene-dicyclopentadiene copolymer, ethylene-propylene-1,5-hexadiene copolymer, and ethyleno-propylene-ethylidene norbornene copolymer; ethylene vinyl compound copolymers such as ethylene-vinyl acetate copolymer, ethylene-vinyl alcohol copolymer, ethylene-vinyl chloride copolymer, ethylene acrylic acid or ethylene-(meth)acrylic acid copolymers, and ethylene-(meth)acrylate copolymer; styrenic copolymers (including elastomers) such as polystyrene, ABS, acrylonitrile-styrene copolymer, $\alpha$-methylstyrene-styrene copolymer, styrene vinyl alcohol, styrene acrylates such as styrene methylacrylate, styrene butyl acrylate, styrene butyl methacrylate, and styrene butadienes and crosslinked styrene polymers; and styrene block copolymers (including elastomers) such as styrene-butadiene copolymer and hydrate thereof, and styrene-isoprenestyrene tri-block copolymer; polyvinyl compounds such as polyvinyl chloride, polyvinylidene chloride, vinyl chloride-vinylidene chloride copolynaer, polymethyl acrylate, and polymethyl methacrylate; polyamides such as nylon 6, nylon 6,6, and nylon 12; thermoplastic polyesters such as polyethylene terephthalate and polybutylene terephtalate; polycarbonate, polyphenylene oxide, and the like; and glassy hydrocarbon-based resins, including poly-dicyclopentadiene polymers and related polymers (copolymers, terpolymers); saturated mono-olefins such as vinyl acetate, vinyl propionate and vinyl butyrate and the like; vinyl esters such as esters of monocarboxylic acids, including methyl acrylate, ethyl acrylate, n-butylacrylate, isobutyl acrylate, dodecyl acrylate, n-octyl acrylate, phenyl acrylate, methyl methacrylate, ethyl methacrylate, and butyl methacrylate and the like; acrylonitrile, methacrylonitrile, acrylamide, mixtures thereof; resins produced by ring opening metathesis and cross metathesis polymerization and the like. These resins may be used either alone or in combinations of two or more

In one particular embodiment, the thermoplastic resin may comprise an alpha-olefin interpolymer of ethylene with a comonomer comprising an alkene, such as 1-octene. The ethylene and octene copolymer may be present alone or in combination with another thermoplastic resin, such as ethylene-acrylic acid copolymer. When present together, the weight ratio between the ethylene and octene copolymer and the ethylene-acrylic acid copolymer may range from 1:10 to 10:1, such as from 3:2 to 2:3. The polymeric resin, such as the ethylene-octene copolymer, may have a crystallinity of less than 50%, such as less than 25%. In some embodiments, the crystallinity of the polymer may range from 5 to 35 percent. In other embodiments, the crystallinity may range from 7 to 20 percent.

Embodiments disclosed herein may also include a polymeric component that may include at least one multi-block olefin interpolymer. Suitable multi-block olefin interpolymers may include those described in, for example, U.S. Provisional Patent Application No. 60/818,911. The term "multi-block copolymer" or "multi-block interpolymers" refers to a polymer comprising two or more chemically distinct regions or segments (referred to as "blocks") preferably joined in a linear manner, that is, a polymer comprising chemically differentiated units which are joined end-to-end with respect to polymerized ethylenic functionality, rather than in pendent or grafted fashion. In certain embodiments, the blocks differ in the amount or type of comonomer incorporated therein, the density, the amount of crystallinity, the crystallize size attributable to a polymer of such composition, the type or degree of tacticity (isotactic or syndiotactic), regio-regularity or regio-irregularity, the amount of branching, including long chain branching or hyper-branching, the homogeneity, or any other chemical or physical property. The multi-block copolymers are characterized by unique distributions of polydispersity index (PDI or $M_w/M_n$), block length distribution, and/or block number distribution due to the unique process making of the copolymers. More specifically, when produced in a continuous process, embodiments of the polymers may possess a PDI ranging from 1.7 to 8; from 1.7 to 3.5 in other embodiments; from 1.7 to 2.5 in other embodiments; and from 1.8 to 2.5 or from 1.8 to 2.1 in yet other embodiments. When produced in a batch or semi-batch process, embodiments of the polymers may possess a PDI ranging from 1.0 to 2.9; from 1.3 to 2.5 in other embodiments; from 1.4 to 2.0 in other embodiments; and from 1.4 to 1.8 in yet other embodiments.

One example of the multi-block olefin interpolymer is an ethylene/$\alpha$-olefin block interpolymer. Another example of the multi-block olefin interpolymer is a propylene/$\alpha$ olefin interpolymer. The following description focuses on the interpolymer as having ethylene as the majority monomer, but applies in a similar fashion to propylene-based multi-block interpolymers with regard to general polymer characteristics.

The ethylene/$\alpha$-olefin multi-block copolymers may comprise ethylene and one or more co-polymerizable $\alpha$-olefin comonomers in polymerized form, characterized by multiple (i.e., two or more) blocks or segments of two or more polymerized monomer units differing in chemical or physical properties (block interpolymer). In some embodiments, the copolymer is a multi-block interpolymer. In some embodiments, the multi-block interpolymer may be represented by the following formula:

$$(AB)_n$$

where n is at least 1, and in various embodiments n is an integer greater than 1, such as 2, 3, 4, 5, 10, 15, 20, 30, 40, 50, 60, 70, 80, 90, 100, or higher; "A" represents a hard block or segment; and "B" represents a soft block or segment. Preferably, A's and B's are linked in a linear fashion, not in a branched or a star fashion. "Hard" segments refer to blocks of polymerized units in which ethylene is present in an amount greater than 95 weight percent in some embodiments, and in other embodiments greater than 98 weight percent. In other words, the comonomer content in the hard segments is less than 5 weight percent in some embodiments, and in other embodiments, less than 2 weight percent of the total weight of the hard segments. In some embodiments, the hard segments comprise all or substantially all ethylene. "Soft" segments, on the other hand, refer to blocks of polymerized units in which the comonomer content is greater than 5 weight percent of the total weight of the soft segments in some embodiments, greater than 8 weight percent, greater than 10 weight percent, or greater than 15 weight percent in various other embodiments. In some embodiments, the comonomers content in the soft segments may be greater than 20 weight percent, greater than 25 eight percent, greater than 30 weight percent, greater than 35 weight percent, greater than 40 weight percent, greater than 45 weight percent, greater than 50 weight percent, or greater than 60 weight percent in various other embodiments.

In some embodiments, A blocks and B blocks are randomly distributed along the polymer chain. In other words, the block copolymers do not have a structure like:

$$AAA—AA\text{-}BBB—BB$$

In other embodiments, the block copolymers do not have a third block. In still other embodiments, neither block A nor block B comprises two or more segments (or sub-blocks), such as a tip segnent.

The multi-block interpolymers may be characterized by an average block index, ABI, ranging from greater than zero to 1.0 and a molecular weight distribution, $M_w/M_n$, greater than 1.3. The average block index, ABI, is the weight average of the block index ("BI") for each of the polymer fractions obtained in preparative TREF from 20°C and 110°C, with an increment of 5°C:

$$ABI = \sum (w_i BI_i)$$

where $BI_i$ is the block index for the $i^{th}$ fraction of the multi-block interpolymer obtained in preparative TREF, and $w_i$ is the weight percentage of the $i^{th}$ fraction.

Similarly, the square root of the second moment about the mean, hereinafter referred to as the second moment weight average block index, may be defined as follows:

$$2^{nd} \text{ moment weight average BI} = \sqrt{\frac{\sum (w_i (BI_i - ABI)^2)}{\frac{(N-1)\sum w_i}{N}}}$$

For each polymer fraction, BI is defined by one of the two following equations (both of which give the same BI value):

$$BI = \frac{1/T_X - 1/T_{XO}}{1/T_A - 1/T_{AB}} \text{ or } BI = -\frac{LnP_X - LnP_{XO}}{LnP_A - LnP_{AB}}$$

where $T_X$ is the analytical temperature rising elution fractionation (ATREF) elution temperature for the $i^{th}$ fraction (preferably expressed in Kelvin), $P_X$ is the ethylene mole fraction for the $i^{th}$ fraction, which may be measured by NMR or IR as described below. $P_{AB}$ is the ethylene mole fraction of the whole ethylene/$\alpha$-olefin interpolymer (before fractionation), which also may be measured by NMR or IR. $T_A$ and $P_A$ are the ATREF elution temperature and the ethylene mole fraction for pure "hard segments" (which refer to the crystalline segments of the interpolymer). As an approximation or for polymers where the "hard segment" composition is unknown, the $T_A$ and $P_A$ values are set to those for high density polyethylene homopolymer.

$T_{AB}$ is the ATREF elution temperature for a random copolymer of the same composition (having an ethylene mole fraction of $P_{AB}$) and molecular weight as the multi-block interpolymer. $T_{AB}$ may be calculated from the mole fraction of ethylene (measured by NMR) using the following equation:

$$Ln\,P_{AB} = \alpha/T_{AB} + \beta$$

where $\alpha$ and $\beta$ are two constants which may be determined by a calibration using a number of well characterized preparative TREF fractions of a broad composition random copolymer and/or well characterized random ethylene co-polymers with narrow composition. It should be noted that $\alpha$ and $\beta$ may vary from instrument to instrument. Moreover, one would need to create an appropriate calibration curve with the polymer composition of interest, using appropriate molecular weight ranges and comonomer type for the preparative TREF fractions and/or random copolymers used to create the calibration. There is a slight molecular weight effect. If the calibration curve is obtained from similar molecular weight ranges, such effect would be essentially negligible. In some embodiments, random ethylene copolymers and/or preparative TREF fractions of random copolymers satisfy the following relationship:

$$Ln\,P = -237.83/T_{ATREF} + 0.639$$

The above calibration equation relates the mole fraction of ethylene, P, to the analytical TREF elution temperature, $T_{ATREF}$, for narrow composition random copolymers and/or preparative TREF fractions of broad composition random copolymers. $T_{XO}$ is the ATREF temperature for a random copolymer of the same composition and having an ethylene mole fraction of $P_X$. $T_{XO}$ may be calculated from $LnP_X = \alpha/T_{XO} + \beta$. Conversely, $P_{XO}$ is the ethylene mole fraction for a random copolymer of the same composition and having an TREF temperature of $T_X$, which may be calculated from $Ln\,P_{XO} = \alpha/T_X + \beta$.

Once the block index (BI) for each preparative TREF fraction is obtained, the weight average block index, ABI, for the whole polymer may be calculated. In some embodiments, ABI is greater than zero but less than 0.4 or from 0.1 to 0.3. In other embodiments, ABI is greater than 0.4 and up to 1.0. In yet other embodiments, ABI should be in the range of from 0.4 to 0.7, from 0.5 to 0.7, or from 0.6 to 0.9. In some embodiments, ABI is in the range of from 0.3 to 0.9, from 0.3 to 0.8, or from 0.3 to 0.7, from 0.3 to 0.6, from 0.3 to 0.5, or from 0.3 to 0.4. In other embodiments, ABI is in the range of from 0.4 to 1.0, from 0.5 to 1.0, or from 0.6 to 1.0, from 0.7 to 1.0, from 0.8 to 1.0, or from 0.9 to 1.0,

Another characteristic of the multi-block interpolymer is that the interpolymer may comprise at least one polymer fraction which may be obtained by preparative TREF, wherein the fraction has a block index greater than 0.1 and up to 1.0 and the polymer having a molecular weight distribution, $M_w/M_n$, greater than 1.3. In some embodiments, the polymer fraction has a block index greater than 0.6 and up to 1.0, greater than 0.7 and up to 1.0, greater than 0.8 and up to 1.0, or greater than 0.9 and up to 1.0. In other embodiments, the polymer fraction has a block index greater than 0.1 and up to 1.0, greater than 0.2 and up to 1.0, greater than 0.3 and up to 1.0, greater than 0.4 and up to 1.0, or greater than 0.4 and up to 1.0. In still other embodiments, the polymer fraction has a block index greater than 0.1 and up to 0.5, greater than 0.2 and up to 0.5, greater than 0.3 and up to 0.5, or greater than 0.4 and up to 0.5. In yet other embodiments, the polymer fraction has a block index greater than 0.2 and up to 0.9, greater than 0.3 and up to 0.8, greater than 0.4 and up to 0.7, or greater than 0.5 and up to 0.6.

Ethylene / $\alpha$-olefin multi-block interpolymers used in embodiments disclosed herein may be interpolymers of ethylene with at least one $C_3$-$C_{20}$ $\alpha$-olefin. The interpolymers may further comprise $C_4$-$C_{18}$ diolefin and/or alkenylbenzene. Suitable unsaturated comonomers useful for polymerising with ethylene include, for example, ethylenically unsaturated monomers, conjugated or non-conjugated dienes, polyenes, alkenylbenzenes, etc. Examples of such comonomers include $C_3$-$C_{20}$ $\alpha$-olefins such as propylene, isobutylene, 1-butene, 1-hexene, 1-pentene, 4-methyl-1-pentene, 1-heptene, 1-octene, 1-nonene, 1-decene, and the like. In certain embodiments, the $\alpha$-olefins may be 1-butene or 1-octene. Other suitable monomers include styrene, halo- or alkyl-substituted styrenes, vinylbenzocyclobutane, 1,4-hexadiene, 1,7-octadiene, and naphthenics (such as cyclopentene, cyclohexene, and cyclooctene, for example).

The multi-block interpolymers disclosed herein may be differentiated from conventional, random copolymers, physical blends of polymers, and block copolymers prepared via sequential monomer addition, fluxional catalysts, and anionic or cationic living polymerization techniques. In particular, compared to a random copolymer of the same monomers and monomer content at equivalent crystallinity or modulus, the interpolymers have better (higher) heat resistance as measured by melting point, higher TMA penetration temperature, higher high-temperature tensile strength, and/or higher high-temperature torsion storage modulus as determined by dynamic mechanical analysis. Properties of infill may benefit from the use of embodiments of the multi-block interpolymers, as compared to a random copolymer containing the same monomers and monomer content, the multi-block interpolymers have lower compression set, particularly at elevated temperatures, lower stress relaxation, higher creep resistance, higher tear strength, higher blocking resistance, faster setup due to higher crystallization (solidification) temperature, higher recovery (particularly at elevated temperatures), better abrasion resistance, higher retractive force, and better oil and filler acceptance.

Other olefin interpolymers include polymers comprising monovinylidene aromatic monomers including styrene, o-methyl styrene, p-methyl styrene, t-butylstyrene, and the like. In particular, interpolymers comprising ethylene and styrene may be used. In other embodiments, copolymers comprising ethylene, styrene and a $C_3$-$C_{20}$ $\alpha$-olefin, optionally comprising a $C_4$-$C_{20}$ diene, may be used.

Suitable non-conjugated diene monomers may include straight chain, branched chain or cyclic hydrocarbon diene having from 6 to 15 carbon atoms. Examples of suitable non-conjugated dienes include, but are not limited to, straight chain acyclic dienes, such as 1,4-hexadiene, 1,6-octadiene, 1,7-octadiene, 1,9-decadiene, branched chain acyclic dienes, such as 5-methyl-1,4-hexadiene; 3,7-dimethyl-1,6-octadiene; 3,7-dimethyl-1,7-octadiene and mixed isomers of dihydromyricene and dihydroocinene, single ring alicyclic dienes, such as 1,3-cyclopentadiene; 1,4-cyclohexadiene; 1,5-cyclooctadiene and 1,5-cyclododecadiene, and multi-ring alicyclic fused and bridged ring dienes, such as tetrahydroindene, methyl tetrahydroindene, dicyclopentadiene, bicyclo-(2,2,1)-hepta-2,5-diene; alkenyl, alkylidene, cycloalkenyl and cycloalkylidene norbornenes, such as 5-methylene-2-norbornene (MNB); 5-propenyl-2-norbornene, 5-isopropylidene-2-norbornene, 5-(4-cyclopentenyl)-2-norbornene, 5-cyclohexylidene-2-norbornene, 5-vinyl-2-norbornene, and norbornadiene. Of the dienes typically used to prepare EPDMs, the particularly preferred dienes are 1,4-hexadiene (HD), 5-ethylidene-2-norbornene (ENB), 5-vinylidene-2-norbornene (VNB), 5-methylene-2-norbornene (MNB), and dicyclopentadiene (DCPD).

One class of desirable polymers that may be used in accordance with embodiments disclosed herein includes elastomeric interpolymers of ethylene, a $C_3$-$C_{20}$ $\alpha$-olefin, especially propylene, and optionally one or more diene monomers. Preferred $\alpha$-olefins for use in this embodiment are designated by the formula $CH_2=CHR^*$, where $R^*$ is a linear or branched alkyl group of from 1 to 12 carbon atoms. Examples of suitable $\alpha$-olefins include, but are not limited to, propylene, isobutylene, 1-butene, 1-pentene, 1-hexene, 4-methyl-l-pentene, and 1-octene. A particularly preferred $\alpha$-olefin is propylene. The propylene based polymers are generally referred to in the art as EP or EPDM polymers. Suitable dienes for use in preparing such polymers, especially multi-block EPDM type polymers include conjugated or non-conjugated, straight or branched chain-, cyclic- or polycyclicdienes comprising from 4 to 20 carbons. Preferred dienes include 1,4-pentadiene, 1,4-hexadiene, 5-ethylidene-2-norbornene, dicyclopentadiene, cyclohexadiene, and 5-butylidene-2-norbornene. A particularly preferred diene is 5-ethylidene-2-norbornene.

As one suitable type of resin, the esterification products of a di- or polycarboxylic acid and a diol comprising a diphenol may be used. These resins are illustrated in U.S. Patent No. 3,590,000. Other specific examples of resins include styrene/methacrylate copolymers, and styrene/butadiene copolymers; suspension polymerized styrene butadienes; polyester resins obtained from the reaction of bisphenol A and propylene oxide followed by the reaction of the resulting product with fumaric acid; and branched polyester resins resulting from the reaction of dimethylterephthalate, 1,3-butanediol, 1,2-propanediol, and pentaerythritol, styrene acrylates, and mixtures thereof.

Further, specific embodiments of the present disclosure may employ ethylene-based polymers, propylene-based polymers, propylene-ethylene copolymers, and styrenic copolymers as one component of a composition. Other embodiments of the present disclosure may use polyester resins, including those containing aliphatic diols such as UNOXOL 3,4 diol, available from The Dow Chemical Company (Midland, MI).

In select embodiments, the thermoplastic resin is formed from ethylene-alpha olefin copolymers or propylene-alpha olefin copolymer. In particular, in select embodiments, the thermoplastic resin includes one or more non-polar polyolefins.

In specific embodiments, polyolefin such as polypropylene, polyethylene, copolymers thereof, and blends thereof, as well as ethylene-propylene-diene terpolymers, may be used. In some embodiments, preferred olefinic polymers include homogeneous polymers, as described in U.S. Patent No. 3,645,992 issued to Elston; high density polyethylene (HDPE), as described in U.S. Patent No. 4,076,698 issued to Anderson; heterogeneously branched linear low density polyethylene (LLDPE); heterogeneously branched ultra low linear density polyethylene (ULDFE); homogeneously branched, linear ethylene/alpha-olefin copolymers; homogeneously branched, substantially linear ethylene/alpha-olefin polymers, which can be prepared, for example, by processes disclosed in U.S.Patent Nos. 5,272,236 and 5,278,272 ; and high pressure, free radical polymerized ethylene polymers and copolymers such as low density polyethylene (LDPE) or ethylene vinyl acetate polymers (EVA).

Polymer compositions, and blends thereof, described in U.S. Patent Nos. 6,566,446, 6,538,070, 6,448,341, 6,316,549, 6,111,023, 5,869,575, 5,844,045, or 5,677,383, may also be suitable in some embodiments. In some embodiments, the blends may include two different Ziegler-Natta polymers. In other embodiments, the blends may include blends of a Ziegler-Natta polymer and a metallocene polymer. In still other embodiments, the polymer used herein may be a blend of two different metallocene polymers. In other embodiments, single site catalyst polymers may be used.

In some embodiments, the polymer is a propylene-based copolymer or interpolymer. In some particular embodiments, the propylene/ethylene copolymer or interpolymer is characterized as having substantially isotactic propylene sequences. The term "substantially isotactic propylene sequences" and similar terms mean that the sequences have an isotactic triad (mm) measured by $^{13}$C NMR of greater than 0.85 in one embodiment; greater than 0.90 in another embodiment; greater than 0.92 in another embodiment; and greater than 0.93 in yet another embodiment. Isotactic triads are well-known in the art and are described in, for example, U.S. Patent No. 5,504,172 and WO 00/01745, which refer to the isotactic sequence in terms of a triad unit in the copolymer molecular chain determined by $^{13}$C NMR spectra.

The olefin polymers, copolymers, interpolymers, and multi-block interpolymers may be functionalized by incorporating at least one functional group in its polymer structure. Exemplary functional groups may include, for example, ethylenically unsaturated mono- and di-functional carboxylic acids, ethylenically unsaturated mono- and di-functional carboxylic acid anhydrides, salts thereof and esters thereof. Such functional groups may be grafted to an olefin polymer, or it may be copolymerized with ethylene and an optional additional comonomer to form an interpolymer of ethylene, the functional comonomer and optionally other comonomer(s). Means for grafting functional groups onto polyethylene are described for example in U.S. Patents Nos. 4,762,890, 4,927,888, and 4,950,541. One particularly useful functional group is maleic anhydride.

The amount of the functional group present in the functional polymer may vary. The functional group may be present in an amount of at least 1.0 weight percent in some embodiments; at least 5 weight percent in other embodiments; and at least 7 weight percent in yet other embodiments. The functional group may be present in an amount less than 40 weight percent in some embodiments; less than 30 weight percent in other embodiments; and less than 25 weight percent in yet other embodiments.

In other particular embodiments, the thermoplastic resin may be ethylene vinyl acetate (EVA) based polymers. In other embodiments, the thermoplastic resin may be ethylene-methyl acrylate (EMA) based polymers. In other particular embodiments, the ethylene-alpha olefin copolymer may be ethylene-butene, ethylene-hexene, or ethylene-octene copolymers or interpolymers. In other particular embodiments, the propylene-alpha olefin copolymer may be a propylene-ethylene or a propylene-ethylene-butene copolymer or interpolymer.

The thermoplastic polymer may have a crystallinity as determined by the observance of at least one endotherm when subjected to standard differential scanning calorimetry (DSC) evaluation. For ethylene-based polymers, a melt index ("MI") determined according to ASTM D1238 at 190°C (375°F) with a 2.16 kg (4.75 lb.) weight of 30 g/10 minutes or less in some embodiments; 25 g/10 minutes or less in other embodiments; 22 g/10 minutes or less in other embodiments; and 18 g/10 minutes or less in yet other embodiments. In other embodiments, ethylene-based polymers may have a melt index (MI) of 0.1 g/10 minutes or greater, 0.25 g/10 minutes or greater in other embodiments; 0.5 g/10 minutes or greater in other embodiments; and 0.75 g/10 minutes or greater in yet other embodiments.

Propylene-based polymers may have a Melt Flow Rate ("MFR") determined according to ASTM D1238 at 230°C (446°F) with a 2.16 kg (4.75 lb.) weight of 85 g/10 minutes or less in some embodiments; 70 g/10 minutes or less in other embodiments; 60 g/10 minutes or less in other embodiments; and 50 g/10 minutes or less in yet other embodiments. In other embodiments, propylene-based polymers may have a melt flow rate (MFR) of 0.25 g/10 minutes or greater; 0.7 g/10 minutes or greater in other embodiments; 1.4 g/10 minutes or greater in other embodiments; and 2 g/10 minutes or greater in yet other embodiments.

Ethylene-based polymers may have a density of 0.845 g/cc or greater in some embodiments; 0.85 g/cc or greater in other embodiments; 0.855 g/cc or greater in other embodiments; and 0.86 g/cc or greater in yet other embodiments. In other embodiments, ethylene-based polymers may have a density of 0.97 g/cc or less; 0.96 g/cc or less in other embodiments; 0.955 g/cc or less in other embodiments; and 0.95 g/cc or less in yet other embodiments.

Propylene-based polymers may comprise 5 percent by weight comonomer or greater in some embodiments. In other embodiments, propylene-based polymers may comprise 7 percent by weight comonomer or greater. In other embodiments, propylene-based polymers may contain 35 percent or less comonomer by weight; 25 percent or less comonomer by weight in yet other embodiments.

One class of thermoplastic polymers useful in various embodiments are copolymers of ethylene and 1-octene or 1-butene, where the ethylene copolymer contains 90 weight percent or less ethylene; 85 weight percent or less ethylene in other embodiments; 50 weight percent or greater ethylene in other embodiments; and 55 weight percent or greater ethylene in yet other embodiments. The ethylene copolymer may contain 1-octene or 1-butene from 10 weight percent or greater in some embodiments; 15 weight percent or greater in other embodiments; 50 weight percent or less in other embodiments; and 45 weight percent or less in yet other embodiments. Each of the above weight percentages are based on the weight of the copolymer. In various embodiments, the ethylene copolymers may have a Melt Index of 0.25 g/10 minutes or greater; 0.5 g/10 minutes or greater in other embodiments; 30 g/10 minutes or less in other embodiments; and 20 g/10 minutes or less in yet other embodiments.

Other polymers useful in embodiments may include copolymers of propylene and ethylene, 1-octene, 1-hexene or 1-butene, where the propylene copolymer contains from 95 weight percent or less propylene; 93 weight percent or less in other embodiments; 65 weight percent or greater in other embodiments; and 75 weight percent or greater in yet other embodiments. The propylene copolymer may contain one or more comonomers, such as ethylene, 1-octene, 1-hexene or 1-butene, from 5 weight percent or greater in some embodiments; 7 weight percent or greater in other embodiments; 35 weight percent or less in other embodiments; and 25 weight percent or less in yet other embodiments. In various embodiments, the propylene copolymers may have a Melt Flow Rate of 0.7 g/10 minutes or greater; 1.4 g/10 minutes or greater in other embodiments; 85 g/10 minutes or less in other embodiments; and 55 g/10 minutes or less in yet other embodiments.

Alternatively, instead of a single polymer, a blend of polymers may be employed that has the physical characteristics described herein. For example, it may be desirable to blend a first polymer with relatively high MI or MFR that is outside the range described, with another of relatively low MI or MFR, so that the combined MI or MFR and the averaged density of the blend fall within the described ranges. A more crystalline alpha-olefin polymer may be combined with one of relatively lower crystallinity, such as one having a significant amount of long chain branching, to provide a blend that has substantially equivalent processing capability in preparing froths and foams described herein. Where reference is made to a "polymer" in this specification, it is understood that blends of olefin polymers with equivalent physical characteristics may be employed with like effect and are considered to fall within our description of the various embodiments.

In certain embodiments, the thermoplastic resin may be an ethylene-octene copolymer or interpolymer having a density between 0.857 and 0.911 g/cc and melt index (190°C with 2.16 kg weight) from 0.1 to 100 g/10 min. In other embodiments, the ethylene-octene copolymers may have a density between 0.863 and 0.902 g/cc and melt index (190°C with 2.16 kg weight) from 0.8 to 35 g/10 min. The ethylene-octene copolymer or interpolymer may incorporate 20-45 percent octene by weight of ethylene and octene.

In certain embodiments, the thermoplastic resin may be a propylene-ethylene copolymer or interpolymer having an ethylene content between 5 and 20% by weight and a melt flow rate (230°C with 2.16 kg weight) from 0.5 to 300 g/10 min. In other embodiments, the propylene-ethylene copolymer or interpolymer may have an ethylene content between 9 and 12 percent by weight and a melt flow rate (230°C with 2.16 kg weight) from 1 to 100 g/10 min.

In certain other embodiments, the thermoplastic resin may be a low density polyethylene having a density between 0.911 and 0.925 g/cc and melt index (190°C with 2.16 kg weight) from 0.1 to 100 g/10 min.

In some embodiments, the thermoplastic resin may have a crystallinity of less than 50 percent. In other embodiments, the crystallinity of the resin may be from 5 to 35 percent. In yet other embodiments, the crystallinity may range from 7 to 20 percent.

In some embodiments, the thermoplastic resin is a semi-crystalline polymer and may have a melting point of less than 110°C. In other embodiments, the melting point may be from 25 to 100°C. In yet other embodiments, the melting point may be between 40 and 85°C.

In some embodiments, the thermoplastic resin is a glassy polymer and may have a glass transition temperature of less than 110°C. In other embodiments, the glass transition temperature may be from 20 to 100°C. In yet other embodiments, the glass transition temperature may be from 50 to 75°C.

In certain embodiments, the thermoplastic resin may have a weight average molecular weight greater than 10,000 g/mole. In other embodiments, the weight average molecular weight may be from 20,000 to 150,000 g/mole; in yet other embodiments, from 50,000 to 100,000 g/mole.

The one or more thermoplastic resins may be contained within the aqueous dispersions described herein in an amount from 1 percent by weight to 96 percent by weight polymer solids. For instance, the thermoplastic resin may be present in the aqueous dispersion in an amount from 10 percent by weight to 60 percent by weight in one embodiment, and 20 percent to 50 percent by weight in another embodiment.

**Polyurethanes**

One embodiment of a polyurethane dispersion may include water and polyurethane and/or a mixture capable of forming polyurethane, such as a polyurethane prepolymer, for example. The polyurethane dispersion may also include one or more additives such as surfactants, wetting agents, and viscosity modifiers. Polyurethane forming materials may include, for example, polyurethane prepolymers that retain some minor isocyanate reactivity for some period of time after being dispersed. Also, the terms polyurethane prepolymer and polyurethane may encompass other types of structures such as, for example, urea groups.

Polyurethanes useful in embodiments disclosed herein may include polyurethanes manufactured from prepolymers based on any organic polyisocyanates, modified polyisocyanates, isocyanate based prepolymers, and mixtures thereof. These may include aliphatic and cycloaliphatic isocyanates, including multifunctional aromatic isocyanates such as 2,4- and 2,6-toluenediisocyanate and the corresponding isomeric mixtures; 4,4'-, 2,4'- and 2,2'-diphenyl-methanediisocyanate (MDI) and the corresponding isomeric mixtures; mixtures of 4,4'-, 2,4'- and 2,2'-diphenylmethanediisocyanates and polyphenyl polyethylene polyisocyanates (PMDI); and mixtures of PMDI and toluene diisocyanates.

In some embodiments, polyurethane polymers useful in embodiments of the composite structure may be prepared by bringing and reacting together an aqueous phase with an isocyanate-terminated prepolymer. The resulting polymer may have a foam or gel structure. Suitable prepolymers are described in, for example WO2004074343 (A1) and WO2005097862 (A1)

In some embodiments, the prepolymer may be the reaction product of a polyether polyol with a stoichiometric excess of an isocyanate mixture. The isocyanate mixture may include methylene diphenylisocyanate, toluene diisocyanate, hexamethylene diisocyanate, isophorone diisoayanate, polymethylene polyphenylisocyanate, carbodiimide or allophonate or uretonimine adducts of methylene diphenylisocyanate and mixtures thereof. Isocyanates used to make up the balance of the composition may include polymethylene polyphenylisocyanate, carbodiimide or allophonate or uretonimine adducts of methylene diphenylisocyanate.

Prepolymer formulations in some embodiments may include a polyol component. Active hydrogen containing compounds used in polyurethane production may include compounds having at least two hydroxyl groups or amine groups. Those compounds are referred to herein as polyols. Representatives of suitable polyols are generally known and are described in such publications as High Polymers, Vol. XVI, "Polyurethanes, Chemistry and Technology" by Saunders and Frisch, Interscience Publishers, New York, Vol. I, pp. 32-42, 44-54 (1962) and Vol. II, pp. 5-6, 198-199 (1964); Organic Polymer Chemistry by K. J. Saunders, Chapman and Hall, London, pp. 323-325 (1973); and Developments in Polyurethane, Vol. I, J. M. Burst, ed., Applied Science Publishers, pp. 1-76 (1978). However, any active hydrogen containing compound may be used. Examples of such materials include those selected from the following classes of compositions, alone or in admixture: (a) alkylene oxide adducts of polyhydroxyalkanes; (b) alkylene oxide adducts of non-reducing sugars and sugar derivatives; (c) alkylene oxide adducts of phosphorus and polyphosphorus acids; and (d) alkylene oxide adducts of polyphenols. Polyols of these types are referred to herein as "base polyols." Examples of alkylene oxide adducts of polyhydroxyalkanes useful herein are adducts of ethylene glycol, propylene glycol, 1,3-dihydroxypropane, 1,4-dihydroxybutane, and 1,6-dihydroxyhexane, glycerol, 1,2,4-trihydroxybutane, 1,2,6-dihydroxyhexane, 1,1,1-trimethylolethane, 1,1,1-trimethylolpropane, pentaerythritol, polycaprolactone, xylitol, arabitol, sorbitol, mannitol. Other useful alkylene oxide adducts include adducts of ethylene diamine, glycerin, piperazine, water, ammonia, 1,2,3,4-tetrahydroxy butane, fructose, sucrose. Also useful are poly(oxypropylene) glycols, triols, tetrols and hexols and any of these that are capped with ethylene oxide. These polyols also include poly(oxypropyleneoxyethylene)polyols. The oxyethylene content may comprise less than 80 weight percent of the total polyol weight in some embodiments, and less than 40 weight percent in other embodiments. The ethylene oxide, when used, may be incorporated in any way along the polymer chain, for example, as internal blocks, terminal blocks, randomly distributed blocks, or any combination thereof.

Polyester polyols may also be used to prepare the polyurethane dispersions. Polyester polyols are generally characterized by repeating ester units which may be aromatic or aliphatic and by the presence of terminal primary or secondary hydroxyl groups, but any polyester terminating in at least two active hydrogen groups may be used. For example, the reaction product of the transesterification of glycols with poly(ethylene terephthalate) may be used to prepare polyurethanes disclosed herein.

The polyisocyanate components of the formulations disclosed herein may be prepared using any organic polyisocyanates, modified polyisocyanates, isocyanate based prepolymers, and mixtures thereof. These may include aliphatic and cycloaliphatic isocyanates, including multifunctional aromatic isocyanates such as 2,4- and 2,6-toluenediisocyanate and the corresponding isomeric mixtures; 4,4'-, 2,4'- and 2,2'-diphenyl-methanediisocyanate (MDI) and the corresponding isomeric mixtures; mixtures of 4,4'-, 2,4'- and 2,2'-diphenylmethanediisocyanates and polyphenyl polymethylene polyisocyanates (PMDI); and mixtures of PMDI and toluene diisocyanates.

The aqueous non-ionic hydrophilic polyurethane dispersions may include the reaction product of a non-ionic hydrophilic prepolymer, water, optionally an external surfactant, and optionally a chain-extending reagent. The hydrophilic prepolymer may include the reaction product of a first component and a second component. The first component may include aromatic polyisocyanate, an aliphatic polyisocyanate, and combinations thereof. The second component may include hydrophilic alkylene oxide polyol, a non-ionic hydrophilic alkylene oxide monol, or a mixture of hydrophilic and hydrophobic alkylene oxide polyols or monols or combinations thereof. The aqueous non-ionic hydrophilic polyurethane dispersion may optionally include one or more surfactants.

Other useful polyurethanes may include those described in PCT Application Publication Nos. WO2005097862A1, WO2004074343A1, and WO2004053223A1, and U.S. Patent Application Publication Nos. 20040109992 and 20050192365.

Other Substrate Components

In some embodiments, substrates may be formed from or may include other polymeric and non-polymeric components, including natural or synthetic materials. Other components may include, for example, polyolefins, such as, polyethylene, polypropylene, polybutylene, and the like; polyesters, such as polyethylene terephthalate, poly(glycolic acid) (PGA), poly(lactic acid) (PLA), poly($\beta$-malic acid) (PMLA), poly($\epsilon$-caprolactone) (PCL), poly(p-dioxanone) (PDS), poly(3-hydroxybutyrate) (PHB), and the like; polyamides, such as nylons (nylon-6, nylon-6,6, nylon-6,12, and others); polyaramids, such as KEVLAR®, NOMEX®, and the like, TEFLON®, and polyester nylons (EP); cellulosic esters; cellulosic ethers; cellulosic nitrates; cellulosic acetates; cellulosic acetate butyrates; ethyl cellulose; regenerated cellulose, such as viscose, rayon, and the like; cotton; flax; silk; hemp; and mixtures thereof. In other embodiments, substrates may include polymers such as ethylene-vinyl acetate (EVA), ethylene/ vinyl alcohol copolymers, polystyrene, impact modified polystyrene, ABS, styrene/butadiene block copolymers and hydrogenated derivatives thereof (SBS and SEBS), and thermoplastic polyurethanes. Suitable polyolefins may include linear or low density polyethylene, polypropylene (including atactic, isotactic, syndiotactic and impact modified versions thereof) and poly (4-methyl-1-pentene). Suitable styrenic polymers may include polystyrene, rubber modified polystyrene (HIPS), styrene/acrylonitrile copolymers (SAN), rubber modified SAN (ABS or AES) and styrene maleic anhydride copolymers.

In other embodiments, substrates may be formed from or may include any natural or synthetic pulp or cellulosic fibers including, but not limited to, nonwoody fibers, such as cotton, abaca, kenaf, sabai grass, flax, esparto grass, straw, jute hemp, bagasse, milkweed floss fibers, and pineapple leaf fibers; and woody fibers such as those obtained from deciduous and coniferous trees, including softwood fibers, such as northern and southern softwood kraft fibers; hardwood fibers, such as eucalyptus, maple, birch, and aspen. Woody fibers may be prepared in high-yield or low-yield forms and can be pulped in any known method, including kraft, sulfite, high-yield pulping methods and other known pulping methods. Pulp and fibers prepared from organosolv pulping methods may also be used, including the fibers and methods disclosed in U.S. Patent No. 4,793,898, issued Dec. 27, 1988 to Laamanen et al.; U.S. Patent No. 4,594,130, issued June 10, 1986 to Chang et al.; and U.S. Patent No. 3,585,104. Useful pulp and fibers may also be produced by anthraquinone pulping, exemplified by U.S. Patent No. 5,595,628 issued Jan. 21, 1997, to Gordon et al. Other examples of useful cellulose-based compositions useful in the present invention include those disclosed in U.S. Patent Nos. 6,837,970, 6,824,650, 6,863,940 and in U.S. Patent Application Nos. US20050192402 and 20040149412 .

Cellulose-based compositions and polymers may also be used, including methylcellulose (*i.e.* METHOCEL), hydroxyethyl cellulose (HEC) (*i.e.* CELLOSIZE), ethylcellulose (*i.e.* ETHOCEL), cationic HEC, and other cellulose derivatives. Polyoxyethylene (such as POLYOX) may also be used in some embodiments. Each of the above indicated trademarked products is available from The Dow Chemical Company, Midland, MI. Other cellulose-based compositions and polymers may also include hydroxypropylmethyl cellulose, hydroxypropyl cellulose, hydroxypropylmethyl cellulose phthalate, hydroxypropylmethyl cellulose acetate succinate, carboxymethylethyl cellulose, cellulose acetate phthalate, polyvinylacetal diethylaminoacetate, aminoalkyl methacrylate copolymer, hydroxypropylmethyl cellulose acetate succinate, methacrylic acid copolymers including methacrylic acid - methyl methacrylate copolymers, cellulose acetate trimellitate (CAT), polyvinyl acetate phthalate, shellac, carboxymethyl cellulose, calcium carboxymethyl cellulose, sodium carboxymethyl cellulose, croscarmellose sodium A-type (Ac-di-sol), starch, crystalline cellulose, hydroxypropyl starch, partly pregelatinized starch, polyvinylpyrrolidone, gelatin, gum arabic, ethyl cellulose, polyvinyl alcohol, pullulan, pregelatinized starch, agar, tragacanth, sodium alginate, propyleneglycol alginate, cellulose derivatives, starch derivatives, pectins, polyacrylates, polyvinyl acetate phthalate, oxidized regenerated cellulose, polyacrylates, modified starches (including water-soluble polymers derived from a starch (e.g., corn starch, potato starch, tapioca starch) such as by acetylation, halogenation, hydrolysis (e.g., such as which an acid), or enzymatic action, or any type of water-soluble modified starch, including but not limited to oxidized, ethoxyolated, cationic, lypophilic and pearl starch, may be used), polyvinyl alcohol, polyethylene glycols, natural and synthetic gums like guar gum, xanthan gum, cellulose gum, acacia gum, polycarbophil, polyolefin oxides such as polyethylene oxide, locust bean gum, bentonite, scheroglucan, polyacrylic acids such as carbopol, polycarbophil, poly(methyl vinyl ether-co-methacrylic acid), poly(2-hydroxyethyl methacrylate), poly(methylmethacrylate), poly(isobutylcyanoacrylate), poly(isohexycyanoacrylate) and polydimethylaminoethyl methacrylate, hydrolytically unstable polyesters containing derivatizable groups, alginate, carrageenan, guar gum derivatives, karaya gum, dextran, hyaluronic acid, pullulan, amylose, high amylose starch, hydroxypropylated high amylose starch, dextrin, pectin, chitin, chitosan, levan, elsinan, collagen, gelatin, zein, gluten, soy protein isolate, polysaccharides, whey protein isolate, and casein. In other embodiments combinations of the above described compositions may be used.

Those having ordinary skill in the art will recognize that the above lists are a non-comprehensive listing of suitable polymers. It will be appreciated that the scope of the present disclosure is restricted by the claims only.

**Dispersion Stabilizing Agent**

Embodiments of the present disclosure use a stabilizing agent to promote the formation of a stable dispersion or emulsion. In selected embodiments, the stabilizing agent may be a surfactant, a polymer (different from the thermoplastic polymers detailed above), or mixtures thereof In other embodiments, the thermoplastic resin may be a self-stabilizer, so that an additional exogenous stabilizing agent may not be necessary. For example, a self-stabilizing system may include a partially hydrolyzed polyester, where by combining polyester with an aqueous base, a polyester resin and surfactant-like stabilizer molecule may be produced. In particular, the dispersion stabilizing agent may be used as a dispersant, a surfactant for frothing the dispersion, or may serve both purposes. In addition, one or more stabilizing agents may be used in combination.

In certain embodiments, the dispersion stabilizing agents used for the polyolefin and polyurethane dispersions herein may be a polar polymer, having a polar group as either a comonomer or grafted monomer. In preferred embodiments, the dispersion stabilizing agent may include one or more polar polyolefins, having a polar group as either a comonomer or grafted monomer. Typical polymers include ethylene-acrylic acid (EAA) and ethylene-methacrylic acid copolymers, such as those available under the trademarks PRIMACOR™ (trademark of The Dow Chemical Company), NUCREL™ (trademark of E.I. DuPont de Nemours), and ESCOR™ (trademark of ExxonMobil) and described in U.S. Patent Nos. 4,599,392, 4,988,781, and 5,938,437. Other suitable polymers include ethylene ethyl acrylate (EEA), ethylene methyl methacrylate (EMMA), and ethylene butyl acrylate (EBA) copolymers. Other ethylene-carboxylic acid copolymer may also be used. Those having ordinary skill in the art will recognize that a number of other useful polymers may also be used.

If the polar group of the polymer is acidic or basic in nature, the dispersion stabilizing polymer may be partially or fully neutralized with a neutralizing agent to form the corresponding salt. The salts may be alkali metal or ammonium salts of the fatty acid, prepared by neutralization of the acid with the corresponding base, e.g., NaOH, KOH, and $NH_4OH$. These salts may be formed in situ in the dispersion step, as described more fully below. In certain embodiments, neutralization of the dispersion stabilizing agent, such as a long chain fatty acid or EAA, may be from 25 to 200% on a molar basis; from 50 to 110% on a molar basis in other embodiments. For example, for EAA, the neutralizing agent is a base, such as ammonium hydroxide or potassium hydroxide, for example. Other neutralizing agents may include lithium hydroxide or sodium hydroxide, for example. Those having ordinary skill in the art will appreciate that the selection of an appropriate neutralizing agent depends on the specific composition formulated, and that such a choice is within the knowledge of those of ordinary skill in the art.

Other dispersion stabilizing agents that may be used in the polyolefin and polyurethane dispersions may include long chain fatty acids or fatty acid salts having from 12 to 60 carbon atoms. In other embodiments, the long chain fatty acid or fatty acid salt may have from 12 to 40 carbon atoms.

Additional dispersion stabilizing agents include cationic surfactants, anionic surfactants, or non-ionic surfactants. Examples of anionic surfactants include sulfonates, carboxylates, and phosphates. Examples of cationic surfactants include quaternary amines. Examples of non-ionic surfactants include block copolymers containing ethylene oxide, propylene oxide, butylene oxide, and silicone surfactants. Surfactants useful as a dispersion stabilizing agent may be either external surfactants or internal surfactants. External surfactants are surfactants that do not become chemically reacted into the polymer during dispersion preparation. Examples of external surfactants useful herein include salts of dodecyl benzene sulfonic acid and lauryl sulfonic acid salt. Internal surfactants are surfactants that do become chemically reacted into the polymer during dispersion preparation. An example of an internal surfactant useful herein includes 2,2-dimethylol propionic acid and its salts or sulfonated polyols neutralized with ammonium chloride. A surfactant may be included in formulations disclosed herein in an amount ranging from 0.01 to 8 parts per 100 parts by weight of polyurethane component.

In particular embodiments, the dispersing agent or stabilizing agent may be used in an amount ranging from greater than zero to 60% by weight based on the amount of thermoplastic resin (or thermoplastic resin mixture) used. With respect to the thermoplastic resin and the dispersion stabilizing agent, in some embodiments, the thermoplastic resin may comprise between 30% to 99% (by weight) of the total amount of polymer and dispersion stabilizing agent in the composition. In other embodiments, the thermoplastic resin may comprise between 50% and 80% (by weight) of the total amount of polymer and dispersion stabilizing agent in the composition. In yet other embodiments, the thermoplastic resins may comprise about 70% (by weight) of the total amount of polymer and dispersion stabilizing agent in the composition. For example, long chain fatty acids or salts thereof may be used from 0.5 to 10% by weight based on the amount of thermoplastic resin. In other embodiments, ethylene-acrylic acid or ethylene-methacrylic acid copolymers may be used in an amount from 0.5 to 60% by weight based on the amount of the thermoplastic resin. In yet other embodiments, sulfonic acid salts may be used in an amount from 0.5 to 10% by weight based on the amount of thermoplastic resin,

Currently most commercially available polyurethane dispersions contain DMPA (depot medroxyprogesterone acetate) as an internal surfactant and can be utilized in this invention. In contrast, a family of polyurethane dispersions which does not contain DMPA, rather incorporating non-ionic modifiers based on ethylene oxide as internal surfactants are equally suitable and may provide other technical and commercial advantages. See for example U.S. Patent No. 6,271,276.

As discussed above, more than one dispersion stabilizing agent may be used, and combinations may be used as a dispersion stabilizing agent and as a frothing surfactant, for example. One of ordinary skill in the an will recognize that the dispersants used to create a relatively stable aqueous dispersion may vary depending on the nature of the thermoplastic resin employed.

**Dispersion Formulations**

Dispersion formulations in accordance with embodiments disclosed herein may include a liquid medium, such as water, a thermoplastic resin, a dispersion stabilizing agent, and optionally frothing surfactants, additives, and fillers. In some embodiments, the aqueous dispersions may include polyolefin and/or polyurethane resin particles ranging in size from 0.02 to 10 $\mu$m (microns) from 0.05 to 5 $\mu$m (microns) in another embodiment; and from 0.1 to 2 $\mu$m (microns) in yet other embodiments.

The thermoplastic resin and the dispersion stabilizing agent may be dispersed in a liquid medium, which in some embodiments is water. In some embodiments, sufficient base is added to neutralize the resultant dispersion to achieve a solution having a pH in the range from 6 to 14. In particular embodiments, sufficient base is added to maintain a pH between 9 to 12. Water content of the dispersion may be controlled so that the combined content of the thermoplastic resin and the dispersion stabilizing agent (solids content) may be between 1% to 74% (by volume). In another embodiment, the solids content may range between 25% to 74% (by volume). In yet another embodiment, the solid content may range between 30% to 50% (without filler, by weight). In yet another embodiment, the solids content may range from 40% to 55% (without filler, by weight).

Dispersions formed in accordance with some embodiments may be characterized in having an average particle size of between 0.02 to 5.0 $\mu$m (microns) In other embodiments, dispersions may have an average particle size from 0.04 to 2.0 $\mu$m (microns) "Average particle size" as used herein refers to the volume-mean particle size. In order to measure the particle size, laser-diffraction techniques may be employed for example. A particle size in this description refers to the diameter of the polymer in the dispersion. For polymer particles that are not spherical, the diameter of the particle is the average of the long and short axes of the particle. Particle sizes can be measured on a Beckman-Coulter LS230 laser-diffraction particle size analyzer or other suitable device.

In a specific embodiment, a thermoplastic resin and a stabilizing agent may be melt-kneaded in an extruder along with water and a neutralizing agent, such as ammonia, potassium hydroxide, or a combination of the two, to form a dispersion. Those having ordinary skill in the art will recognize that a number of other neutralizing agents may be used. In some embodiments, filler may be added before, during, or after blending the thermoplastic resin and stabilizing agent.

Any melt-kneading means known in the art may be used. In some embodiments, a kneader, a BANBURY® mixer, single-screw extruder, or a multiscrew extruder is used. A process for producing the dispersions in accordance with the present disclosure is not particularly limited. Processes for melt-kneading the above-mentioned components are disclosed in U.S. Patent No. 5,756,659 and U.S. Patent Publication No. 20010011118, for example.

Figure 2 schematically illustrates an extrusion apparatus that may be used in embodiments of the disclosure. An extruder 20, in certain embodiments a twin screw extruder, is coupled to a back pressure regulator, melt pump, or gear pump 30. Embodiments also provide a base reservoir 40 and an initial water reservoir 50, each of which includes a pump (not shown). Desired amounts of base and initial water are provided from the base reservoir 40 and the initial water reservoir 50, respectively. Any suitable pump may be used, but in some embodiments a pump that provides a flow of about 150 cc/min at a pressure of 240 bar is used to provide the base and the initial water to the extruder 20. In other embodiments, a liquid injection pump provides a flow of 300 cc/min at 200 bar or 600 cc/min at 133 bar. In some embodiments, the base and initial water are preheated in a preheater.

Generally, any method known to one skilled in the art of preparing thermoplastic polymer (polyolefin and polyurethane) dispersions may be used. A suitable storage-stable polyurethane dispersion as defined herein is any polyurethane dispersions having a mean particle size of less than 5 μm (microns) Polyurethane dispersions that are not storage-stable may have a mean particle size of greater than 5 microns. For example, a suitable dispersion may be prepared by mixing a polyurethane prepolymer with water and dispersing the prepolymer in the water using a mixer. Alternatively, a suitable dispersion may be prepared by feeding a prepolymer into a static mixing device along with water, and dispersing the water and prepolymer in the static mixer. Continuous methods for preparing aqueous dispersions of polyurethane are known and may be used in embodiments disclosed herein, For example, U.S. Patent Nos. 4,857,565, 4,742,095, 4,879,322, 3,437,624, 5,037,864, 5,221,710, 4,237,264, and 4,092,286 all describe continuous processes useful for preparing polyurethane dispersions. In addition, a polyurethane dispersion having a high internal phase ratio can be prepared by a continuous process such as is . described in U.S. Patent No. 5,539,021.

Other types of aqueous polymer dispersions may be used in combination with the polyolefin and polyurethane dispersions useful in embodiments disclosed herein. Suitable dispersions useful for blending with polyurethane dispersions include: styrene-butadiene dispersions; styrene-butadiene-vinylidene chloride dispersions; styrene-alkyl acrylate dispersions; ethylene vinyl acetate dispersions; polychloropropylene latexes; polyethylene copolymer latexes; ethylene styrene copolymer latexes; polyvinyl chloride latexes; or acrylic dispersions, like compounds, and mixtures thereof.

In producing embodiments of the polyurethane dispersions, the surfactants may be added to the polyurethane dispersion along with antioxidants, bactericides, etc., when viscosity is low and good mixing may be obtained. The dispersion stabilizing agent may then be added followed by any inorganic filler, slowly enough to ensure good dispersion and avoid clumping/lumping of the filler. Finally the thickener may be added to obtain the desired viscosity. It is believed that the addition of ammonium stearate after the filler and thickener addition avoids swelling of the polyurethane dispersion particle, resulting in a lower viscosity during mixing.

**Frothing Surfactants**

Surfactants useful for preparing froths are referred to herein as frothing surfactants. A frothing surfactant allows the gas, commonly air, used in frothing to disperse homogenously and efficiently into the formulated foamed dispersion. Preferably, the frothing surfactant produces a non-sudsing composite foam product after drying.

Embodiments of the present disclosure may use a frothing surfactant to promote the formation of a stable dispersion and to aid in frothing. Creating and stabilizing the froth during the frothing and drying steps may be accomplished by addition of a froth stabilizing surfactant to the aqueous dispersion of the polyolefin resin when initially creating the froth. In addition, these surfactants may also be used to improve aqueous wetting of dried foams, if desired. Suitable frothing surfactants may be selected from cationic, nonionic and anionic surfactants: In some embodiments, frothing surfactants may include the stabilizing agents as described above.

In some embodiments, the frothing surfactant may be an alkylcellulose ethers, hydroxyalkyl cellulose ethers, hydroxyalkyl alkylcellulose ethers, guar gum, xanthan gum, and polyoxyethylene resins of at least 20,000 molecular weight, or combinations thereof. Other suitable frothing surfactants may be selected from cationic surfactants, anionic surfactants, or non-ionic surfactants. Examples of cationic surfactants include quaternary amines, primary amine salts, diamine salts, and ethoxylated amines. Examples of non-ionic surfactants include block copolymers containing ethylene oxide, silicone surfactants, alkylphenol ethoxylates, and linear and secondary alcohol ethoxylates of alkyl group containing more than 8 carbon atoms.

Examples of cationic surfactants include quaternary amines, primary amine salts, diamine salts, and ethoxylated amines. Examples of non-ionic surfactants include block copolymers containing ethylene oxide, silicone surfactants, alkylphenol ethoxylates, and linear and secondary alcohol ethoxylates of alkyl group containing more than 8 carbon atoms.

Examples of anionic surfactants include sulfonates, carboxylates, and phosphates. In one embodiment, anionic surfactants useful in preparing the froth from the aqueous dispersion may be selected from carboxylic acid salts and ester amides of carboxylic fatty acids, preferably fatty acids comprising from 12-36 carbon atoms, e.g., stearic or lauric acid, palmitic, myristic, oleic, linoleic, ricinoleic, erucic acid and the like.

In some embodiments, the surfactant may include amphoteric surfactants such as aminopropionates, amphoteric sulfonates, betaines, imidazoline based amphoterics, and sultaines, among others. For example, the surfactant may be derived from an imidazoline and can either be the acetate form (containing salt) or the propionate form (salt-free). Examples of suitable amphoteric surfactants include surfactants such as lauramidopropyl betaine, sodium laurimino dipropionate, cocoamidopropyl hydroxyl sultaine, alkylether hydroxypropyl sultaine, sodium capryloampho hydroxypropyl sulfonate, disodium capryloampho dipropionate, sodium cocoamphoacetate, disodium cocoamphodiacetate, sodium cocoamphopropionate, disodium octyl iminodipropionate, sodium cocoampho hydroxypropyl sulfonate, disodium lauryl iminodipropionate, sodium stearoampho acetate, and disodium tallow iminodipropionate, among others. Other amphoteric surfactants known in the art may also be used.

In one embodiment, when a good "hand" or fabric-like feel is desired in the finished foam, a saturated fatty acid derivative (e.g., the salt of stearic or palmitic acid) may be used. Other suitable anionic surfactants include alkylbenzene sulfonates, secondary n-alkane sulfonates, alpha-olefin sulfonates, dialkyl diphenylene oxide sulfonates, sulfosuccinate esters, isothionates, linear alkyl (alcohol) sulfates and linear alcohol ether sulfates. It is understood that the froth stabilizing surfactants may or may not be different than those used to prepare the dispersion. These surfactants serve both to assist in froth formation and help to stabilize the froth. In a particular embodiment, the surfactant may be selected from at least one of alkali metal, mono-, di- and tri-alkanol amine (mono-, di- or triethanol amine, for example), and ammonium salts of lauryl sulfate, dodecylbenzene sulfates, alcohol ethoxy sulfates, and isothionates, the dibasic salt of N-octyldecylsulfosuccinimate, and mixtures thereof. In other embodiments, the froth stabilizing agent may include cellulose.

In some embodiments, the frothing surfactant may be used in an amount such that the resulting froth, as described below, may contain from 0.01 to 10.0 weight percent frothing surfactant based on the dry weight of the thermoplastic polymer. In other embodiments, the froth may contain from 0.02 to 3.0 weight percent frothing surfactant based on the dry weight of the thermoplastic polymer; from 0.03 to 2.5 weight percent based on the dry weight of the thermoplastic polymer in other embodiments; and from 0.05 to 10.0 weight percent based on the dry weight of the thermoplastic polymer in yet other embodiments. In various other embodiments, the frothing surfactant may be present in the froth in an amount ranging from a lower bound of 0.01, 0.02, 0.03, 0.04, or 0.05 weight percent based on the dry weight of the thermoplastic polymer to an upper bound of 2.0, 2.5, 3.0, 4.0, 5.0, or 10.0 weight percent based on the dry weight of the thermoplastic polymer, in any combination of given upper and lower bounds.

In addition to the above listed surfactants, other surfactants may be used which do not detrimentally affect the frothing or stability of the froth. In particular additional anionic, zwitterionic, or nonionic surfactants may be used in combination with the above listed surfactants.

**Additives**

The polymers, dispersions, froths, and foams disclosed herein may optionally contain fillers in amounts, depending on the application for which they are designed, ranging from 2-100 percent (dry basis) of the weight of the thermoplastic resin. These optional ingredients may include, for example, calcium carbonate, titanium dioxide powder, polymer particles, hollow glass spheres, fibrillated fibers, polymeric fibers such as polyolefin based staple monofilaments and the like. Foams designed for use in the absorbent articles may contain bulk liquid-absorbing material, such as short cotton fiber or other cellulose fiber evenly distributed throughout the polymer foam.

Additives may be used with the thermoplastic polymers, dispersion stabilizing agents, frothing surfactants, or fillers without deviating from the scope of the present disclosure. For example, additives may include a wetting agent, surfactants, antistatic agents, antifoam agent, anti block, wax-dispersion pigments, a neutralizing agent, a thickener, a compatibilizer, a brightener, a rheology modifier, a biocide, a fungicide, and other additives known to those skilled in the art.

Additives and adjuvants may be included in any formulation comprising the thermoplastic polymers. Suitable additives include fillers, such as organic or inorganic particles, including clays, talc, titanium dioxide, zeolites, powdered metals, organic or inorganic fibers, including carbon fibers, silicon nitride fibers, steel wire or mesh, and nylon or polyester cording, nano-sized particles, clays, and so forth; tackifiers, oil extenders, including paraffinic or napthelenic oils; and other natural and synthetic polymers, including other polymers according to embodiments of the invention.

The compositions disclosed herein may contain processing oils, plasticizers, and processing aids (collectively referred to as processing oils). Processing oils having a certain ASTM designation and paraffinic, napthenic or aromatic process oils are all suitable for use. In some embodiments, from 0 to 150 parts processing oils per 100 parts of total polymer may be employed; from 0 to 100 parts in other embodiments; and from 0 to 50 parts of oil per 100 parts of total polymer are employed in yet other embodiments. Higher amounts of processing oil may tend to improve the processing of the resulting product at the expense of some physical properties. Additional processing aids include conventional waxes, fatty acid salts, such as calcium stearate or zinc stearate, (poly)alcohols including glycols, (poly)alcohol ethers, including glycol ethers, (poly)esters, including (poly)glycol esters, and metal salts, especially Group 1 or 2 metal salts or zinc salt derivatives thereof.

Compositions, including thermoplastic blends, may also contain anti-ozonants or anti-oxidants. The anti-ozonants may be physical protectants such as waxy materials that come to the surface and protect the thermoplastic from oxygen or ozone or they may be chemical protectors that react with oxygen or ozone. Suitable chemical protectors include styrenated phenols, butylated octylated phenol, butylated di(dimethylbenzyl) phenol, p-phenylenediamines, butylated reaction products of p-cresol and dicyclopentadiene (DCPD), polyphenolic anitioxidants, hydroquinone derivatives, quinoline, diphenylene antioxidants, thioester antioxidants, and blends thereof. Some representative trade names of such products are WINGSTAY™ S antioxidant, POLYSTAY™ 100 antioxidant, POLYSTAY™ 100 AZ antioxidant, POLYSTAY™ 200 antioxidant, WINGSTAY™ L antioxidant, WINGSTAY™ LHLS antioxidant, WINGSTAY™ K antioxidant, WINGSTAY™ 29 antioxidant, WINGSTAY™ SN-1 antioxidant, and IRGANOX™ antioxidants. In some applications, the anti-oxidants and anti-ozonants used will preferably be non-staining and non-migratory.

For providing additional stability against UV radiation, hindered amine light stabilizers (HALS) and UV absorbers may be also used. Suitable examples include TINUVIN™ 123, TINUVIN™ 144, TINUVIN™ 622, TINUVIN™ 765, TINUVIN™ 770, and TINUVIN™ 780, available from Ciba Specialty Chemicals, and CHEMISORB™ T944, available from Cytex Plastics, Houston TX, USA. A Lewis acid may be additionally included with a HALS compound in order to achieve superior surface quality, as disclosed in U.S. Patent No. 6,051,681.

For some compositions, additional mixing processes may be employed to predisperse the anti-oxidants, anti-ozonants, carbon black, UV absorbers, and/or light stabilizers to form a masterbatch, and subsequently to form polymer blends there from.

Suitable crosslinking agents (also referred to as curing or vulcanizing agents) for use herein include sulfur based, peroxide based, or phenolic based compounds. Examples of the foregoing materials are found in the art, including in U.S. Patents No.: 3,758,643, 3,806,558, 5,051,478, 4,104,210, 4,130,535, 4,202,801, 4,271,049, 4,340,684, 4,250,273, 4,927,882, 4,311,628 and 5,248,729.

Other prepolymers may also include a chain extender or crosslinker. A chain extender may be used to build the molecular weight of the polyurethane prepolymer by reaction of the chain extender with the isocyanate functionality in the polyurethane prepolymer, that is, chain extend the polyurethane prepolymer. A suitable chain extender or crosslinker is typically a low equivalent weight active hydrogen containing compound having 2 or more active hydrogen groups per molecule. Chain extenders typically have 2 or more active hydrogen groups while crosslinkers have 3 or more active hydrogen groups. The active hydrogen groups may be hydroxyl, mercaptyl, or amino groups. An amine chain extender may be blocked, encapsulated, or otherwise rendered less reactive. Other materials, particularly water, may function to extend chain length and, therefore, may be chain extenders.

Polyamines chain extenders may be selected prom amine terminated polyethers such as, for example, JEFFAMINE® D-400 from Huntsman Chemical Company, aminoethyl piperazine, 2-methyl piperazine, 1,5-diamino-3-methylpentane, isophorone diamine, ethylene diamine, diethylene triamine, aminoethyl ethanolamine, triethylene tetraamine, triethylene pentaamine, ethanol amine, lysine in any of its stereoisomeric forms and salts thereof, hexane diamine, hydrazine and piperazine. In some embodiments, the chain extender may be used as an aqueous solution.

In the formation of polyurethane dispersions, a chain extender may be employed in an amount sufficient to react with from zero to 100 percent of the isocyanate functionality present in the prepolyxner, based on one equivalent of isocyanate reacting with one equivalent of chain extender. It may be desirable to allow water to act as a chain extender and react with some or all of the isocyanate functionality present. A catalyst may optionally be used to promote the reaction between a chain extender and an isocyanate. When chain extenders have more than two active hydrogen groups, they may also concurrently function as crosslinkers.

Thermoplastic compositions according to embodiments of the invention may also contain organic or inorganic fillers or other additives such as starch, talc, calcium carbonate, glass fibers, polymeric fibers (including nylon, rayon, cotton, polyester, and polyaramide), metal fibers, flakes or particles, expandable layered silicates, phosphates or carbonates, such as clays, mica, silica, alumina, aluminosilicates or aluminophosphates, carbon whiskers, carbon fibers, nanoparticles including nanotubes, wollastonite, graphite, zeolites, and ceramics, such as silicon carbide, silicon nitride or titania. Silane based or other coupling agents may also be employed for better filler bonding.

Examples of conventional fillers include milled glass, calcium carbonate, aluminum trihydrate, talc, bentonite, antimony trioxide, kaolin, fly ash, or other known fillers. A suitable filler loading, in a polyurethane dispersion for example, may be from 0 to 200 parts of filler per 100 parts of dispersion solids (pphds). Fillers may be loaded in an amount of less than 100 pphds in some embodiments, and less than 80 pphds in other embodiments. Addition of inorganic fillers may enhance the production of the foam composite by faster drying speeds on the production line because the percentage of water to be removed on drying is lower.

Optionally a filler wetting agent may be used. A filler wetting agent may improve the compatibility of the filler and the polyolefin or polyurethane dispersions. Useful wetting agents include phosphate salts such as sodium hexametaphosphate. A filler wetting agent may be included at a concentration of at least 0.5 pphds.

Thickeners may be useful to increase the viscosity of polyurethane and polyolefin dispersions. For example, suitable thickeners include ALCOGUM™ VEP-II (a trade designation of Alco Chemical Corporation) and PARAGUM™ 241 (a trade designation of Para-Chem Southern, Inc.). Other suitable thickeners may include cellulose derivatives such as METHOCEL™ products (a trade designation of The Dow Chemical Company). Thickeners may be used in any amount necessary to prepare a dispersion of desired viscosity.

Thickening agents may be used when it is desired to control the viscosity of the aqueous phase and facilitate the transportation and distribution of, for example, fillers or fibers. Fillers may include clays, diatomaceous earth, calcium carbonate, and mineral fibers such as wallastonite; aqueous, latexes such as for example a styrene-butadiene. Thickening agents may include natural products such as xanthan gums, or chemical agents such as polyacrylamide polymers and gels. Other additives include mixing aids and emulsifiers.

The aqueous phase may also be used to introduce to other substances, such as fatty oils and functional additives, besides fibers and fillers when desiring to modify physical properties of the resulting polymer. Also present may be fragrances or perfumes or other such substances that can be detected by scent should this be required for the end application. If the end application requires a polymer that has some physiological active properties, the aqueous phase can also be used to introduce active molecules such as for example, pesticides, insecticides, herbicides, attractants, pheromones, growth promoting or regulating substances or plant or animal nutrients. If the resulting polymer is to be used in end applications where electrical or luminescent properties are required, the aqueous mixture may be used to introduce electrolytes so as to render the polymer electro-conductive, or fluorescent or phosphorescent additives so as to render the polymer luminescent. While generally such additional substances are introduced via the aqueous phase, the isocyanate-terminated prepolymer can also be utilized in the same manner when no adverse reactions or process conditions prevail.

While optional for purposes of the present invention, some components can be highly advantageous for product stability and durability during and after the manufacturing process. For example, inclusion of antioxidants, biocides, and preservatives may be highly advantageous in some embodiments.

**Froth Preparation**

For preparing froths from the above described dispersions, a gaseous frothing agent is generally used. Examples of suitable frothing agents include: gases and/or mixtures of gases such as, for example, air, carbon dioxide, nitrogen, argon, helium. Frothing agents are typically introduced by introduction of a gas above atmospheric pressure into a dispersion to form a homogeneous froth by mechanical shear forces during a predetermined residence time. In preparing froths, all components of the dispersion may be mixed and then the gas may be blended into the mixture, using equipment such as an OAKES™, COWIE & RIDING™, or FIRESTONE® frother.

Froths may be prepared from the dispersion/surfactant/optional additives mixtures by using a mechanical method such as a high shear, mechanical mixing process under atmospheric conditions to entrain air or other gases in the aqueous phase of the dispersion or optionally injecting gas into the system while mixing. The amount of air or other gas (where a gas in addition to or other than air is desirable) that may be incorporated in the froth may comprise at least 80% by volume in one embodiment, at least 85% by volume in another embodiment, and at least 90% by volume of the resultant froth in yet another embodiment. Initially, all components to be used in making the froth may be mixed together with mild agitation to avoid entrapping air.

Once all of the ingredients are well mixed, the mixture may be exposed to high shear mechanical mixing. During this step, the bulk viscosity of the mixture may increase as more air is entrapped within the continuous aqueous phase until a non-flowable, stiff froth is formed. The mixing time necessary to obtain a froth with the desired density may vary with amount and type of froth stabilizing surfactant and the amount of mechanical shear. Any mechanical mixing device capable of whipping air into a thickened aqueous dispersion, such as a kitchen blender/hand mixer, Hobart mixer fitted with a wire whip, or, on a larger scale, a COWIE-RIDING™ Twin Foamer (Cowie Riding Ltd.) may be used. The commercial foamers may also allow one to inject air into their high shear mixing head to obtain very low (less than 50 g/L) density froth.

Froth density may be measured, for example, by drawing off samples of the froth in cups of predetermined volume and weight, weighing the froth-filled cup, and then calculating the density of the sample. In commercial frothers, air can be added directly into the mixing head to assist in development of low density froth. The speed of the frothing device may be increased or decreased to attain a desired froth density. In one embodiment, the froth density may be in a range of 0.04 to 0.15 g/cc, and from 0.07 to 0.10 g/cc in another embodiment. In other embodiments, the froth density may be in from 0.05 g/cc to 0.09 g/cc. Once a desired density of the froth is obtained, the froth may be optionally spread on a substrate prior to conversion of the froth into foam.

Frothed foams comprising the polymers may also be formed as disclosed in PCT Application PCT/US2004/027593, filed August 25, 2004, and published as WO2005/021622. In other embodiments, the polymers may also be crosslinked, preferably after forming the foam, by any known means, such as the use of peroxide, electron beam, silane, azide, gamma irradiation, ultraviolet radiation, or other crosslinking techniques. The polymers may also be chemically modified, such as by grafting (for example by use of maleic anhydride (MAH), silanes, or other grafting agent), halogenation, amination, sulfonation, or other chemical modification.

Drying and Recovery Steps

In one embodiment, the foam may be prepared from the froth by removing at least a portion of the liquid/aqueous element of the froth prepared as disclosed herein. In other embodiments, the foam may be prepared from the froth by removing at least a majority of the liquid/aqueous element of the froth. In yet other embodiments, the foam may be prepared by removing substantially all of the liquid/aqueous element. In various embodiments, greater than 30 weight percent, greater than 50 weight percent, greater than 80 weight percent, greater than 90 weight percent, greater than 95 weight percent, greater than 98 weight percent, or greater than 99 weight percent of the liquid/aqueous element may be removed. The means by which the liquid portion is removed may be selected to minimize the amount of froth volume collapse. In one embodiment, the froths may be dried and converted to foams by heating in a forced air drying oven, at temperatures selected for optimum drying. In one embodiment, the froth may be heated to a temperature between 60°C and 120°C.

As the nature of the thermoplastic resin permits, processing may be conducted at the highest temperature feasible to remove water as rapidly as possible from the froth without destroying the viscosity of the polyolefin resin particles on the surface of the bubbles of the froth or causing significant (e.g., more than 75-80 volume percent) collapse of the partially dried froth. In another embodiment, the drying temperature may be selected so as to not exceed, the melting point temperature of the thermoplastic fibers. In one embodiment, it may be desirable to dry the froth at a temperature that approaches, but does not exceed the melting range of the thermoplastic resin. In another embodiment, it may be desirable to attain a temperature where the amorphous regions in the thermoplastic resin begin to coalesce while pseudo-crosslinking with the fibers and avoid or at least minimize collapse of the froth before the foam has become fully "dried" in its ultimate form and dimension and at least 95 weight percent of the water in the froth has been driven out. The resulting "dried" foam may has a density of 0.03 to 0.07 g/cc ; and from about 0.03 to 0.05 g/ce in other embodiments. In other embodiments, the foams may have a density within the range of 0.03 g/cc to 0.06 g/cc.

Some embodiments of the dried foam may have an average thickness ranging from 0.5 mm to 300 mm or more; from 1 mm to 6 mm in other embodiments; and from 0.01 cm to 2.5 cm in yet other embodiments. Other embodiments of the dried foam may have an average thickness ranging from 0.05 cm to 2.0 cm; and from 1 to 1.5 cm in yet other embodiments. Articles comprising embodiments of the dried foam may include at least one layer of foam having an average thickness ranging from 0.1 cm to 2.5 cm; from 0.5 cm to 2.0 cm in other embodiments; and from 1.0 cm to 1.5 cm in yet other embodiments. In some embodiments, two or more foams may be laminated together; in various embodiments, the two or more foams may have the same or different densities, the same or different cell sizes, or the same or different structures (fibrillated, open-celled, closed-celled, etc.). Open-cell foams have interconnected pores or cells, whereas closed-cell foams do not have interconnected pores or cells. In other embodiments, one or more foams may be laminated to a substrate, such as film.

Drying of the froth to form the desired foam of the disclosure may be conducted in batch or continuous mode. Devices including, for example, conventional forced air drying ovens or banks of infrared heating lamps or dielectric heating devices, e.g., radio (typically operated at permitted frequency bands in the range between 1-100 MHz) and microwave (typically operated at permitted frequency bands in the range between 400 to 2500 MHz) frequency energy generating sources, lining a tunnel or chamber in which the froth may be placed or conveyed through, in a continuous fashion, may be employed for drying. A combination of such drying energy sources may be used, either simultaneously or sequentially applied, to dry a froth to form a foam. In one embodiment, the drying includes the simultaneous use of a dielectric device and a forced air drying oven. For foam having a thickness of 0.25 to 0.6 cm, the drying may be achieved as quickly as 45 to 90 seconds when the forced air oven is operated at approximately 75°C and a radio frequency generator heats the froth to an internal temperature of 45 to 50°C. The temperature of the drying operation may be selected according to the nature and the melting range of the polyolefin resin (as determined by DSC) employed to prepare the foam. The dielectric heating frequency bands, permitted for industrial use in various countries, are designated in greater detail in the reference "Foundations of Industrial Applications of Microware and Radio Frequency Fields," Rousy, G and Pierce, J. A. (1995).

In one embodiment, the absorbent structure (the foam) may have a non-cellular, fibrillated morphology. As used herein, a "non-cellular, fibrillated structure" refers to a foam having an open, random, non-cellular, morphology composed of or having fibrils or thread-like filaments. The non-cellular, fibrillated structure, for example, may be non-uniform and non-repeating, such as where the fibrils form a non-woven fibrous-like web and where a majority of the struts are not interconnected.

In other embodiments, the foams disclosed herein may be open-cell foams, wherein the cell size of the majority of cells of the foam ranges between 5 and 1000 micrometers. In other embodiments, the foam may be characterized by having a majority of its cells being substantially ellipsoidal in shape.

The foams described herein may adhere to a substrate. In some embodiments, the adhesive force between the foam and the substrate may be 17.5 N/m (0.1 lb$_f$/in) or greater. In other embodiments, the adhesive force between the foam and the substrate may be 26.3N/m (0.15 lb$_f$/in) or greater; and 32N/m (0.2 lb$_f$/in) or greater in yet other embodiments.

**TEXTILE SUBSTRATE**

Substrates that may be used in embodiments of the composite structures disclosed herein may include non-wovens, elastic non-wovens, and soft non-wovens. Non-woven textiles are those that are neither woven nor knit. Non-wovcns are typically manufactured by putting small fibers together in the form of a sheet or web, and then binding them mechanically, with an adhesive, or thermally. In other embodiments, substrates may include fabrics or other textiles, porous films, and other non-wovens, including coated substrates. In certain embodiments, the substrate may be a soft textile, such as a soft or elastic non-woven, such as an elastomeric polyolefin or a polyurethane, for example. Wovens and/or knits made from microdenier fibers may also provided the desired substrate performance.

In some embodiments, the non-wovens may be based on polyolefin monocomponent fibers, such as polyethylene. In other embodiments, bicomponent fibers may be used, for example where the core is based on a polypropylene and the sheath may be based on polyethylene. It should be understood that the fibers used in embodiments of the composite structure may be continuous or non-continuous, such as staple fibers.

One example of a suitable soft non-woven is described in WO20051111282A1, disclosing a non-woven material having a fuzz/abrasion resistance of less than 0.5 mg/cm2, and a flexural rigidity of less than or equal to 0.043 * Basis Weight - 0.657 mN-cm. The non-woven material may have a basis weight greater than 15 grams/m$^2$, a machine direction (MD) tensile strength of more than 25 N/5cm in MD (at a basis weight of 20 grams/m$^2$), and a consolidation area of less than 25%. In other embodiments, a spun-bond non-woven fabric may be made using fibers having a diameter in a range of from 0.1 to 50 denier.

An additional specific example of a suitable soft non-woven is described in WO2005111291A1, disclosing a non-woven material having a fuzz/abrasion resistance of less than 0.7 mg/cm2 and a flexural rigidity of less than 0.15 mN.cm. The non-woven material may have a basis weight greater than 15 grams/m$^2$, a tensile strength of more than 10 N/5cm MD and 7 N/5cm cross direction (CD) (at a basis weight of 20 GSM), and a consolidation area of less than 25%. In other embodiments, a fiber from 0,1 to 50 denier may be formed from a polymer blend, wherein the polymer blend includes a) from 40 weight percent to 80 weight percent by weight of the polymer blend of a first polymer which is a homogeneous ethylenela/$\alpha$-olefin interpolymer having: 1) a melt index of from 1 to 1000 grams/10 minutes, and 2) a density of from 0.87 to 0.95 grams/cc, and b) from 60 to 20 percent by weight of a second polymer which may be an ethylene homopolymer or an ethylene/a-olefin interpolymer having: 1) a melt index of from 1 to 1000 grams/10 minutes, and 2) a density which is at least 0.01 grams/cc greater than the density of the first polymer. Homogeneous ethylene/ $\alpha$-olefin polymers are those having a composition distribution breadth index (CDBI) of at least 70% in some embodiments, at least 80% in other embodiments, and as high as 100% in yet other embodiments. CDBI is defined herein as described in U.S. Patent No, 5,246,783 and WO 93/04486, using the apparatus described in U.S, Patent No. 5,008,204.

Additionally, a web having similar physical properties to those described above may also be utilized. The web structure may be formed from individual fibers, filaments, or threads which are interlaid, but not in an identifiable manner. Non-woven fabrics or webs have been formed from many processes such as melt blowing, spun-bonding, electrospun, and bonded carded web processes. The basis weight of the non-wovens ranges from 15 to 250 g/m$^2$ in some embodiments; 20 to 250 g/m$^2$ in other embodiments .

In some embodiments, elastic non-wovens, such as described in U.S. Patent No 6,994,763 may be used. The elastic non-woven may be based on bicomponent fibers, where the core component may an elastomeric polymer and the sheath component may a polyolefin. Representative examples of commercially available elastomers for the core component of the bicomponent fiber may include the following polymers: KRATON® Polymers, ENGAGE™ polymers, VERSIFY™ elastomers, INFUSE™ olefin block copolymers, VISTAMAXX™ polyolefin elastomers, VECTOR™ polymers, polyurethane elastomeric materials ("TPU"), and heterophasic block copolymers. Representative materials for the sheath component may include polyolefin based homo- and co- polymers. The polyolefin polymers may include polypropylene homopolymer, polypropylene random copolymers, polypropylene impact copolymers, and polyethylenes. In some embodiments, the polyethylenes may have a density ranging from 0.925g/cm$^3$ to 0.965g/cm$^3$.

In other embodiments, suitable elastic non-wovens may be formed from one or more "elastomeric" polymers. The term "elastomeric" generally refers to polymers that, when subjected to an elongation, deform or stretch within their elastic limit. For example, spun-bonded fabrics formed from elastomeric filaments typically have a root mean square average recoverable elongation of at least 75% based on machine direction and cross direction recoverable elongation values of the fabric after 30% elongation of the fabric and one pull. Advantageously, spun-bonded fabrics formed from elastomeric filaments typically have a root mean square average recoverable elongation of at least 65% based on machine direction and cross direction recoverable elongation values of the fabric after 50% elongation of the fabric and one pull.

In some embodiments, substrates include non-woven fibrous webs. In other embodiments, the above mentioned substrates may be coated with the above mentioned polyolefin and polyurethane dispersions to form polyolefin- or polyurethane-coated substrates useful in embodiments of the composite structures disclosed herein.

In other embodiments, apertured films may be utilized as a layer(s) of the composite structures or laminates described herein. Use of apertured films may increase the strength of the structure. Additionally the apertured films may provide a suitable feel for applications not requiring contact with the face or other sensitive skin.

Descriptions of apertured films may be found in WO200080341A1 and U.S. Patent Nos. 3,929,135 and 4,324,246. Apertured films may include thin polymeric films with small openings space uniformly across the width of the film. Apertured films are commonly used for use in body contacting absorbent and non-absorbent articles such as baby diapers, adult incontinent articles, sanitary napkins or panty liners, facial wipes, body wipes, articles of clothing, hospital bed sheets and the like.

In other embodiments, the composite structures described herein may include substrates coated with the polyurethane dispersions and/or polyolefin dispersions, such as described above, or similar coatings for increased durability of the substrate and improved softness.

Non-woven substrates used in the composite structures may include mono- and bi- component fibers having a basis weight ranging from 15 to 2509 g/m$^2$ in some embodiments; 20 to 250 g/m$^2$ in other embodiments; and a basis weight ranging from 25 to 60 g/m$^2$ in other embodiments. Basis weight may be determined by measuring the weight of a known area of fabric. For example, basis weight may be determined according to ASTM D 3776.

In other embodiments, the composite structures described herein may be formed from a substrate having an abrasion loss of less than 0.7 mg/cm$^2$; less than 0.6 mg/cm$^2$ in other embodiments; and less than 0.5 mg/cm$^2$ in yet other embodiments. Abrasion loss, or the amount of fuzz generated during abrasion, may be measured by the rub test.

The rub test is performed by rubbing sandpaper of a defined grit across the surface of the sample with a controlled force. The sample is weighed before and after the test and weight loss is measured to determine the amount of fuzz formed and removed from the surface by the sandpaper.

ARTICLES

Composite structures formed from one or more of the above described substrates, including foam and fabric substrates may have a balance of softness, weight, and other properties which may include bending rigidity, coefficient of friction, fuzz resistance, loft, volume, and others.

Composite structures include at least one substrate layer and at least one open-cell foam layer. The substrate includes non-wovens, fabrics, and the like. Incorporation of open-cell foams with a substrate (e.g., non-wovens, fabrics, etc.) into wipes or other articles may impart additional softness, loft, and volume to the article. The additional loft and volume may be achieved while enhancing and/or maintaining the desired, pre-existing surface feel, of the substrate alone. The incorporation of the open-cell foam may also increase the available void volume and/or surface area for the inclusion and delivery of active agents when compared to the fabric or non-woven layer alone.

In other embodiments, composite structures may include an open-cell foam layer, a substrate layer, and optionally at least one cleaning surfactant, active agent, or enhancing filler. Embodiments of the composite structure may exhibit a desired combination of performance properties, including high softness and high loft, and/or excellent resistance to surface abrasion. The soft, high loft composite structure may be useful for disposable and semi-disposable applications related to personal care, medical, shipping and household markets. The composite structure may also be capable of delivering wet active agents or dry active agents requiring wetting for cleansing, polishing or medical applications.

In other embodiments, the composite structure may include an impervious substrate layer. For example, a thermoplastic film or thermoplastic impregnated paper layer may provide a barrier between layers of the composite structure. Impervious substrate layers may be advantageously used where one side of the structure is used for delivering wet or dry active agents, and where a second side is to remain free of the wet or dry active agents. For example, EP0951228 discloses use of an impervious layer in a pad having handles that may be used for application of fingernail polish remover (acetone) while limiting skin contact with the polish remover. Impervious layers may also be advantageously used where the formation of a wet/dry wipe is desired.

The composite structures disclosed herein may be used for cleaning wipes for skin contact, and may include wet and/or dry active agents. The composite structures disclosed herein may also be used for other applications including baby wipes, hand wipes, hard surface cleaners for home use, and industrial cleaning wipes.

Enhanced softness or a more cloth-like feel are also desirable for applications beyond skin cleansing wipes. These applications may include, but are not limited to, applicator pads, polishing cloths, medical cleansing, shipping/ packaging material for sensitive components, and an application pad for topical medicines. Additionally these articles may be used as a means for the temporary storage of measured amounts liquid materials.

The above foams and/or coatings (dispersions and/or froths) may be used in combination with the substrates (foams and/or fabrics) also defined above. The following process/technologies may be used in the manufacture of these various combinations. Methods described below are typical and detailed descriptions of the techniques can be found in standard texts.

In one embodiment, a substrate (foams and/or fabrics) may be contacted with a fluid, such as an active compound described below, to form a composite structure. The resulting composite structure may have desired properties, such as basis weight, rigidity, coefficient of friction, and fuzz resistance.

In some embodiments, the composite structures may be formed by extrusion coating. The froth, such as a polyolefin froth may be extruded directly onto the desired substrate. A second substrate may be applied to the top of the froth or foam. Layers may be repeated as necessary. The sandwiched composite may then be heated to dry the foam and to adhere the layers together.

In other embodiments, the composite structures may be formed by roll coating (doctor blade). The wet foam or froth may be applied to a continuous belt of substrate using a doctor blade a fixed height above the substrate. Wet foam continuously fed to one side of the blade creates a constant pool of material. The moving substrate below the blade pulls from this pool of material with the thickness of the resultant coating to be fixed by the blade height. Additional layers of substrate or foam may be added as required. The resultant structure may then be dried to remove moisture and to aid in adhesion.

In other embodiments, the composite structures may be formed by adhesive lamination. The dried foam may be adhesively laminated onto the desired substrate

In other embodiments, the composite structures may be formed by spray coating. The froth or wet foam may be sprayed onto desired substrate and subsequently dried.

In other embodiments, the composite structures may be formed by curtain coating. Wet foam or froth may be applied via direct deposition onto a moving belt or substrate. The coating thickness is controlled by the froth or wet foam feed rate and the speed of the substrate below coating curtain.

In other embodiments, the composite structures may be formed by batch application. The froth or wet foam may be manually applied to a substrate surface. The surface may then be leveled using a knife blade and metering bars of desired thickness. The knife moves across the metering bars removing wet foam from the surface creating a uniform height.

In other embodiments, it may be desirable to form an article having two or more foam layers. In some embodiments, the foam layers may be of the same or different density. In other embodiments the two or more foam layers may be laminated.

Additional processing techniques may include thermoforming; embossing, hydroentaglement, air lacing, exposure to infrared heat, and addition of surface fibers, such as flocking techniques.

The resulting composite structures may have one or more desired physical properties. In some embodiments, the resulting composite structures may have an advantageous combination of the desired physical properties.

The composite structure is formed from substrates having a basis weight of 15 to 250 g/m$^2$. In other embodiments, the composite structure may be formed from substrates having a basis weight of 20 to 250 g/m$^2$; from 20 to 80 g/m$^2$; and from 25 to 50 g/m$^2$ in yet other embodiments. Basis weight may be determined by measuring the weight of a known area of fabric, where the area is no smaller than 50 mm$^2$. For example, basis weight may be determined according to ASTM D 3776.

In some embodiments, composite structures may be formed by incorporating a liquid, such as an active, with a foam substrate. In other embodiments, composite structures may include one or more foam layers. Foams and foam substrates may have a thickness of from 0.5 to 300 mm in some embodiments, and from 1 to 6 mm in yet other embodiments. Polyolefin foams and foam substrates may have a density from 0.025 to 0.1 g/cc in some embodiments, and from 0.03 to 0.06 g/cc in other embodiments. Polyurethane foams and foam substrates may have a density from 0.025 g/cc to 0.5 g/cc in some embodiments, and from 0.05 to 0.1 g/cc in other embodiments. Foam density may be measured by weighing a dried foam sample of known dimensions (volume).

In some embodiments, the composite structures may have a Kawabata Evaluation System (KES) coefficient of friction ranging from 0.1 to 1.0 MIU. In other embodiments, the composite structures may have a KES coefficient of friction ranging from 0.3 to 1.0 MIU; from 0.4 to 0.9 MIU in other embodiments; and from 0.5 to 0.8 MIU in yet other embodiments. The composite structure may have a KES surface roughness, in either the machine direction or the cross direction, of less than 4.0 μm (microns) in some embodiments. In other embodiments, the composite structure may have a KES surface roughness of less than 3.5 μm (microns); less than 3.25 μm (microns) in other embodiments; and less than 3.0 μm (microns) in yet other embodiments. Surface properties (resistance / drag / friction) and surface contour (roughness) values are as determined using a KES-FB4 surface tester, where a tension load of 20 gf/cm is applied to the sample. MIU is a measure of the coefficient of friction on a scale of 0 to 1, where a higher MIU value corresponds to a greater friction or resistance and drag. Regarding KES surface roughness, measured in microns, a higher value corresponds to a geometrically rougher surface.

In some embodiments, the composite structures may have Kawabata Evaluation System (KES) compression resilience from 30% to 50%. In other embodiments, the composite structures may have a KES compression resilience ranging from 35% to 45%; and from 37% to 43% in yet other embodiments. KES compression resilience is as determined using a KES-FB3 compression tester, measured by applying a varied load from 0-10 gf/cm$^2$ to a 2 cm$^2$ area of the samples. Compression resilience is the percent recovery of the material thickness after the applied load is removed. Higher compression resilience numbers are favorable and indicate a greater percent recovery after being compressed.

The composite structures may have a bending (flexural) rigidity of less than 1000 mN·cm in some embodiments, for both the cross direction and machine direction. In other embodiments, the composite structures may have a bending rigidity of less than 900 mN·cm; less than 800 mN·cm in other embodiments; and less than 700 mN·cm in yet other embodiments. Bending rigidity is a measure of the energy required to bend the fabric to a 41.5° decline, where the energy is given per unit fabric width. To measure bending rigidity, the substrate sample is cut into a piece of 25.4 mm by 152.4 mm and is set onto a raised platform allowing a 41.5° angle below the horizontal of the platform to be formed. The sample is then moved forward at a constant rate off the platform until the unsupported end of the sample flexes to contact the 41.5° angle. The bending length is one half the measured sample overhang length. Additionally, the sample basis weight is calculated as outlined above. The bending rigidity is calculated by $G = m \times C^3\ 10^{-3}$ (mN-cm), where m is the basis weight of the sample (g/m$^2$) and C is the bending length of the sample in cm. The acceleration due to gravity (9.81 m/s$^2$) has been rounded to 10 m/s$^2$ in determining this equation. The bending rigidity of the non-woven fabric was also measured in both the longitudinal direction and the transverse direction.

In some embodiments, the composite structures may have a machine direction tensile strength of greater than 10 N/5cm; greater than 12 N/5cm in other embodiments; and greater than 15 N/5cm in yet other embodiments. Tensile strength of a fabric may be measured, for example, using test method DIN53354. Tensile strength is measured by cutting the fabric into a piece 5 cm by 10 cm, where the long edge is in the machine direction, holding the fabric in the chucks of a tensile tester and measuring for tensile strength at a pull rate of 100 mm/min.

In some embodiments, the composite structures may have a cross direction tensile strength of greater than 10 N/5cm; greater than 12 N/5cm in other embodiments; and greater than 15 N/5cm in yet other embodiments. Tensile strength of a fabric may be measured, for example, using test method DIN53354. Tensile strength is measured by cutting the fabric into a piece 5 cm by 10 cm, where the long edge is in the cross direction, holding the fabric in the chucks of a tensile tester and measuring for tensile strength at a pull rate of 100 mm/min.

In some embodiments, the composite structure may have a good fuzz resistance. The composite may have a Rub Test fuzz generation of 0.7 $mg/cm^2$ or less in some embodiments, 0.6 $mg/cm^2$ in other embodiments; and 0.5 $mg/cm^2$ in yet other embodiments. The rub test fuzz generation is a weight measure of fibers per unit area removed from a sample after abrasion with a rough surface. To determine fuzz generation, an 11 cm by 4 cm sample is weighed, placed into a Sutherland Ink Rub Tester. A piece of 320 grit sandpaper of suitable size is adhered to a 2 pound weighted holder within the tester. The weighted holder is then placed onto the specimen, and the rub tester is then started with a speed of 42 cycles per minute. The tester is then run for a total of 20 cycles. The weighted holder is then removed from the sample. Any loose fibers remaining on the specimen surface are removed with gentle application and removal of adhesive tape. The weight of the sample after abrasion is then recorded. The fuzz generation value is calculated as the weight loss of the sample per unit area of the sample. Lower fuzz generation values are considered desirable.

In some embodiments, the composite structure may have a good "hand" quality. The composite structure may have a "hand" value, normalized for sample volume, of 25 $gf/cm^3$ or less in some embodiments; 14 $gf/cm^3$ or less in other embodiments; 13 $gf/cm^3$ or less in other embodiments; and 11 $gf/cm^3$ or less in yet other embodiments. Additionally, the composite structures may have a "hand" quality, normalized for sample weight, of 220 gf/g or less in some embodiments; 160 gf/g or less in other embodiments; 158 gf/g or less in other embodiments; and 118 gf/g or less in yet other embodiments. The "hand" quality is considered to be the combination of resistance due to surface friction, flexibility, and compressibility of a fabric material. As described in INDA 1st 90.3 (95), a Handle-O-Meter tester (manufactured by Thwing-Albert Instrument Co., West Berlin, NJ) measures the above factors using a Linear Variable Differential Transformer (LVDT) to detect the resistance that a blade encounters when forcing a specimen of material into a slot of parallel edges. A 3/2 digital voltmeter (DVM) indicates the resistance directly in grams. Samples of the composites were cut into three 50$cm^2$ circle specimens and were conditioned at 50% relative humidity and 21.1 °C (70°F) prior to testing. The Handle-O-Meter slot width was set at 20 mm. Measurements were taken in each of four positions per specimen as required by the instrument manufacturer's test manual, and the four measurements were summed to give the total hand for a single specimen in grams-force, with the total hand being averaged for the three specimens. This averaged hand was then normalized to the specimen weight and volume.

In some embodiments, the composite structure may have a balance of softness and strength. For example, a composite structure formed by contacting a fluid, such as an active, with a foam or fabric substrate may have a dry basis weight of 25 to 150 $g/m^2$, a flexural rigidity of less than 1000 mN·cm, and a coefficient of friction within the range from 0.4 to 0.9 MIU. In other embodiments, the composite structure may also have a Rub Test fuzz level of 0.7 $mg/cm^2$ or less. And in other embodiments, the composite structure may also have a surface roughness of less than 3.5 SMD. In yet other embodiments, the composite structure may also have a hand of less then 25 $gf/cm^3$

Where the composite structure includes two or more layers, the layers may be adhered together with or without adhesives In some embodiments, the adhesion of a first layer to an adjoining second layer may be 17.5 cm N/m (0.1 $lb_f$/in) or greater. In other embodiments, the adhesive strength may be 26.3 N/m ($lb_f$/in) or greater; 32 N/m (0.2 $lb_f$/in) or greater in yet other embodiments. Adhesive strength, or laminate peel strength, is a measure of the energy required to separate the layers per unit area. Adhesive strength is the average load per unit width of bond line required to part bonded materials, where the angle of separation is 180 degrees and separation rate is 6 in/min (ASTM D-903).

ACTIVE AGENTS

The composite structures described using the above foam technologies, substrates and application techniques may also include wet or dry active agents for increased performance within specific end use applications. Active agents for direct or post introduction may include: additives for cleaning, such as surfactants, enhancing fillers (such as exfoliates for abrasion or skin peeling additives), alcohols or oils; wellness additives such as oils moisturizing agents; additives for skin cleaning like make-up removing oils; additives which help to recover stressed skin, like zinc salts, camomile, marigold, aloe vera, vitamins and minerals; skin care actives, such as proteins (hydrolized collagen, elastin, keratin, soy, corn, wheat, oats, silk, and synthetic peptides, for example), botanicals (green tea, grape seed, and the like), polysaccharides (such as hyaluronic acid, phycopolysaccharides, b-1, 3-glucans, chitosan), and enzymes (bromelain, papain, superoxide dismutase, subtilisin). Additional active agents may include moisture/odor absorbing compounds such as silica gel, activated carbon, zeolites, etc.

These active agents, such as surfactants, emollients, soaps, inorganics, moisturizers, fragrance, cleaner, antimicrobial, vitamins and fillers may be introduced into the structure via multiple mechanisms. Incorporation of actives may be achieved using some or all of the techniques described below.

In other embodiments, the active agent may be added to a polyolefin dispersion prior to frothing. In other embodiments, the active agent may be added to the composite structure, either to the surface of the foam or into the pores of the foam, via post-treatment or standard coating techniques. In other embodiments, the active agent may be added to a non-woven substrate using standard coating techniques. In yet other embodiments, the active agent may be added as an additional layer or a pocket within the composite structure.

EXAMPLES

Example 1

A first substrate layer of DREAMEX® (a polyethylene copolymer based elastic non-woven having a basis weight of 50 g/m$^2$, available from Corovin GmbH Corporation, Germany) is placed on silicone release paper. A template measuring 200 mm x 400 mm x 3.0 mm is then placed on the substrate.

A froth formed from an AFFINITY™ based dispersion is prepared by the following procedure. An aqueous dispersion of a polyolefm plastomer is formed in accordance with the procedures as described in W02005021638. The dispersion is formed using AFFINITY™ EG 8200 (an ethylene-alpha olefin copolymer having an MI2 of about 5 dg/min, and a density of about 0.87 g/cc). The dispersion surfactant system includes UNICID® 350 (a linear carboxylic acid available from Baker Petrolite), HYSTRENE® 4516 (a fatty acid available from Crompton Corp., Greenwich, Connecticut), and METHOCEL® (a water-soluble methylcellulose and hydroxypropyl methylcellulose polymer available from The Dow Chemical Company), used at a loading of 2 weight percent, 1 weight percent, and 0.35 weight percent, respectively, based on the combined weight of the ethylene copolymer and the surfactant system. The aqueous dispersion produced has a solids content of approximately 53 weight percent: To produce a foam, the above described dispersion is then mixed with 1 weight percent sodium lauryl sulfate (STEPANOL® WAT-K, available from Stepan Co.), based on polymer solids.

Approximately 160 grams of the aqueous dispersion is added to a mixing bowl. The dispersion is mixed using a Hobart bench top mixer on high setting for 3 minutes, targeting a froth density of 0.06g/cc. The froth is then spread evenly over the template and substrate using a spatula, and the template removed. A second substrate layer of DREAMEX™ is then placed on top of the just applied froth. The composite structure is then placed in an oven at 75°C for 25-30 minutes or until surface temperature reaches 70-75°C. The sample is then cooled to room temperature and cut into 50 cm$^2$ sample disks using a circular die.

Example 2

The procedures of Example 1 are repeated with a 200 mm x 400 mm x 4.4 mm template.

Example 3

The procedures of Example 1 are repeated with a 200 mm x 400 mm x 5.5 mm template.

Example 4

The procedures of Example 1 are repeated with a 200 mm x 400 mm x 4.4 mm template, and the mixing is performed using a Hobart bench top mixer on high setting for 1.5 minutes, targeting a froth density of 0.091 g/cc.

Example 5

The procedures of Example 1 are repeated with a 200 mm x 400 mm x 5.5 mm template, the first substrate is a polyethylene copolymer based elastic non-woven having a basis weight of 50 g/m$^2$, and the second substrate is a polyethylene copolymer based elastic non-woven having a basis weight of 25 g/m$^2$.

Example 6

The procedures of Example 1 were repeated with a 200 mm x 400 mm x 4.4 mm template; the mixing is performed using a Hobart bench top mixer on high setting for 1.5 minutes, targeting a froth density of 0.091 g/cc; the first substrate is a polyethylene copolymer based elastic non-woven having a basis weight of 50 g/m$^2$; and the second substrate is a polyethylene copolymer based elastic non-woven having a basis weight of 25 g/m$^2$.

Example 7

The procedures of Example 1 were repeated with a 200 mm x 400 mm x 4.4 mm template, the first substrate is a polyethylene copolymer based soft non-woven having a basis weight of 19.3 g/m$^2$, and the second substrate is a polyethylene copolymer based soft non-woven having a basis weight of 19.3 g/m$^2$.

Wipes samples with cleaning agent

A cleaning agent is prepared by mixing components as follows. The first component includes 225 grams de-ionized water and 6 grams Stepanquat ML (Quaternium 82). The second component includes 6 grams Ciba Salicare SC-95 (Polyquaternium-37, Mineral Oil, PPG-1 Trideceth-6), 60 grams Stepan Octyl Isonanoate (ethylhexyl isononanoate), and 3 grams Goldschmidt Abil EM-9 (cetyl dimethicone copolyol). The third component includes 0.03 grams Herbal lavender Q-12672, a fragrance, and 1.2 grams Lonza Glydant, a preservative. Component 1 is added to Component 2, and is mixed for about 20 minutes, after which Component 3 is added and the solution is homogenized.

Example 8

The cleaning agent is added to **Example 3** to meet a cleaning agent to substrate volume of 0.15 g cleaning agent per 1 cm$^3$ substrate.

Example 9

The cleaning agent is added to **Example 5** to meet a cleaning agent to substrate volume of 0.15 g cleaning agent per 1 cm$^3$ substrate.

Example 10

The cleaning agent is added to Example 7 to meet a cleaning agent to substrate volume of 0.15 g cleaning

agent per 1 cm$^3$ substrate.

**Example 11**

**A** soft non-woven substrate is formed using the AFFINITY™ based 2-1-1 dispersion described above diluted with deionized water to yield about 5% solids content (approximately 90 grams water to 10 grams POD dispersion). A soft non-woven (Haberer/386 ELITE 5600) utilizing a polyethylene-based copolymer and having a basis weight of 20 g/m$^2$ is submerged in the diluted dispersion, removed, and is allowed to air dry for 24 hrs, resulting in a polyolefin coated substrate having a dry coat weight of approximately 0.22 grams solid per gram non-woven.

**A** first substrate layer of the polyolefin coated soft non-woven substrate is placed on silicone release paper. 4.7 mm thickness bars are then placed 300 mm apart on the substrate that was placed on release paper.

**A** froth formed from the AFFINITY™ based dispersion is prepared by the following procedure. Approximately 100 grams of the dispersion is added to a mixing bowl. The dispersion is mixed using a Hobart bench top mixer on high setting for 3 minutes, targeting a froth density of 0.095g/cc. The froth is then spread evenly over the substrate using a spatula, and the bars removed. A second substrate layer of the polyolefin coated non-woven is then placed on top of the just applied froth. The composite structure is then placed in an oven at 75°C for 25-30 minutes or until surface temperature reaches 70-75°C. The sample is then cooled to room temperature and cut into 50 cm$^2$ sample disks using a circular die.

**Example 12**

**A** hydrophilic aqueous poly(urea/urethane) dispersion (HYPOL), available from The Dow Chemical Company, Midland, Michigan), prepared using a continuous mechanical dispersion process such as described in U.S. Patent Nos. 5,339,021, 5,688,842, 5,959,027, and 6,087,440, is diluted with deionized water to yield about 5% solids content (approximately 75 grams water to 25 grams poly(urea/urethane) dispersion). A soft non-woven (Haberer/386 ELITE 5600) utilizing a polyethylene-based copolymer and having a basis weight of 20 g/m$^2$ is submerged in the dilute dispersion, removed, and is allowed to air dry for 24 hrs, resulting in a polyurethane coated substrate having a dry coat weight of approximately 0.27 grams solid per gram non-woven.

**A** first substrate layer of the polyurethane coated soft non-woven substrate is placed on silicone release paper. 4.7 mm thickness bars are then placed 300 mm apart on the substrate that was placed on release paper.

**A** froth formed from an AFFINITY™ based dispersion is prepared by the following procedure. Approximately 100 grams of the dispersion is added to a mixing bowl. The dispersion is mixed using a Hobart bench top mixer on high setting for 3 minutes, targeting a froth density of 0.095g/cc. The froth is then spread evenly over the substrate using a spatula, and the bars removed. A second substrate layer of the polyurethane coated non-woven is then placed on top of the just applied froth. The composite structure is then placed in an oven at 75°C for 25-30 minutes or until surface temperature reaches 70-75°C. The sample is then cooled to room temperature and cut into 50 cm$^2$ sample disks using a circular die.

**Example 13**

**Polyolefin** foam samples are made by frothing 150 grams of the AFFINITY™ based 2-1-1 dispersion described above. The dispersion is mixed for 3 minutes on high using a Kitchen Aid mixer utilizing a Hobart brand wire whisk, resulting in a froth having a density of 0.10 g/cc. The resulting froth is applied to silicon release paper using a 20.2 cm x 20.2 cm x 0.5 cm template. The shaped froth layer is then dried for 30 minutes at 75°C.

**The** resulting foam is then cut into 10.16 cm x 10.16 cm (4" x 4") samples and is placed onto spunbond, hydrophilic, PGI non-woven substrate. An additional layer of non-woven is placed on top of the foam. The edges of the samples are then crimped using a heat sealer, forming a square pouch with foam on the interior.

**In** one sample, the non-woven is heat crimped directly to the interior foam layer, in another example, the non-woven is heat crimped around the foam on the interior. In this latter sample, the foam is not adhered to the non-woven but is encased by the non-woven. In the earlier sample the foam is bound to the non-woven around the perimeter with the center of the foam not being adhered to the non-woven.

**Example 14**

**The** procedures of Example 1 are repeated with a 200 mm x 400 mm x 4.4 mm template, where the first substrate is a polyethylene copolymer based elastic non-woven having a basis weight of 50 g/m$^2$, and the second substrate is a polyethylene copolymer based elastic non-woven having a basis weight of 25 g/m$^2$.

**Comparison Products**

**Products** existing in the marketplace were identified and compared to samples generated in Examples 1-16. The five identified products are referenced as Comparative Samples 1-5. Specifically, the products are: Comparative Sample 1) NEUTROGENA® Pure Glow Daily Cleansing Cushion; Comparative Sample 2) OLAY® Daily Facials Deep Cleansing Cloths; Comparative Sample 3) PONDS® Clean Sweep Cleansing & Makeup Removing Towlettes; Comparative Sample 4) DOVE® Gentle Exfoliating Daily Facial Cleansing Pillows; and Comparative Sample 5) BIROE™ Pore Perfect Daily Deep Pore Cleansing Cloths.

**Comparative** Samples and samples generated from the above described examples were tested for physical properties. The resulting data is discussed in three sections below: Sensory Panel Testing, Physical Property Tests, and Kawabata Evaluation System (KES) Testing. The various test methods provide a mechanism to describe the advantages that the composite structures disclosed herein have over existing products.

**Sensory Panel Testing**

Sensory Panel Testing provides a system of forced ranking to compare the Examples to the Comparative Samples by human touch. Within these data the following attributes were compared; hand friction, depression depth, fullness/volume, stiffness, and thermal. These attributes are important to the perception of softness. It is desirable to provide a product that is low in hand friction, high in depression depth, high in fullness/volume, low in stiffness and high in thermal (or "warmest feeling").

These results show that the dry Examples are higher in depression depth, high fullness/volume, lower in warmth, and comparable to higher in hand friction than the Comparative Samples. The results also show that the Examples are low in stiffness compared to the bulkier, higher volume Comparative Samples.

These trends hold for Examples that have a cleaning solution incorporated i.e. wet samples with the exception of the warmth and hand friction. These attributes shift to being comparable in both attributes to the wet Comparative Samples. The data, for wet and dry samples, show that, overall, the Examples have more desirable softness attributes then the Comparative Samples, especially balance of loft and flexibility.

A human sensory panel was used to evaluate article attributes believed to be associated with the perception of softness. The attributes are defined as follows. Twenty-four trained panelists evaluated the samples, with a random order of presentation, and random three-digit labeling for the samples. The samples were evaluated for hand friction or slipperiness, depression depth, fullness and volume, stiffness, thermal characteristics.

Hand friction is evaluated by moving one's hand across the surface of the composite structure. The sample is placed flat on a table, evaluation side up. Using the weight of the hand and forearm, the hand is moved horizontally across the surface in all four directions parallel to the edges. Slipperiness is evaluated based on a no drag to drag scale from 1 to 6, where 1 equals the least amount of drag (most slip), and 6 equals the most drag.

Depression depth is characterized by evaluating the amount of the sample depression when downward force is applied. The sample is placed flat on the table. Finger tips are used to press down gently on the center of the sample. The downward force is then released. Depression depth is evaluated based on a 1 to 6 scale, where 1 equals the least amount of depression and 6 equals the most depression.

Fullness and volume are evaluated based upon the amount of material sample felt in the hand during manipulation. The sample is placed flat on the table. Then, one's dominant hand is placed on top of the sample, positioned so the fingers are pointing toward the top of the sample. The fingers are then closed, gathering the composite sample with fingers toward palm. The non-dominant hand is then used to press the sample into the cupped dominant hand. The dominant hand is then closed slightly and the sample manipulated by rotating the sample in the palm. Fullness and volume are also measured on a scale from 1 to 6, where 1 is the least full (low volume), and 6 is the most full (high volume).

Stiffness evaluates the degree to which the sample feels pointed, ridged, or cracked. The sample is placed flat, and one's dominant hand is placed on top of the sample; position so the fingers are pointing toward the top of the sample. The fingers are then closed, gathering the composite sample with fingers toward palm. The non-dominant hand is then used to press the sample into the cupped dominant hand. The dominant hand is then closed slightly and the sample manipulated by rotating the sample in the palm. Stiffness is also measured on a scale from 1 to 6, where 1 is the least stiff (most pliable), and 6 is the stiffest (least pliable).

Thermal characteristics evaluate the difference in the heating or cooling effect felt when the sample is placed against skin. The sample is held flat in the palm of one's hand. One's other hand is placed on top of the hand, and the temperature effect evaluated. Thermal characteristics are evaluated on a scale of 1 to 6, where 1 is the coolest feel, and 6 is the wannest feel

The samples evaluated are shown in Table 2, along with the panel results (averaged). Depression depth, fullness/volume, and stiffness were evaluated on the above mentioned scales. The sample thickness was quantitatively measured with applied force of 0.00035 Mpa (0.05 psi) onto 2.54 cm (1") diameter area using a linear gauge. These thickness measurements are included for reference.

Table 2.

| Sample | Sample Description | Sample Thickness (mm) | Hand Friction | Depression Depth | Fullness/ Volume | Stiffness |
|---|---|---|---|---|---|---|
| Example 2 | Elastic nonwoven, Low Density PFF | 3.1 | 3.75 | 3.13 | 3.13 | 2.75 |

(continued)

| Sample | Sample Description | Sample Thickness (mm) | Hand Friction | Depression Depth | Fullness/ Volume | Stiffness |
|--------|-------------------|----------------------|---------------|------------------|------------------|-----------|
| Example 4 | Elastic nonwoven, High Density PFF | 3.5 | 3.63 | 3.38 | 3.96 | 4.54 |
| Example 1 | Elastic nonwoven, Low Density PFF | 1.9 | 3.58 | 1.29 | 1.54 | 2.33 |
| Example 3 | Elastic nonwoven, Low Density PFF | 4.3 | 2.83 | 4.83 | 4.63 | 2.63 |
| Example 7 | Soft nonwoven, Low Density PFF | 1.3 | 1.21 | 2.38 | 1.75 | 2.75 |

The elastic non-woven (Examples 1, 2, 3, and 4) resulted in increased hand friction (more drag) (>2.8) feel than the soft non-woven (1.2). Thicker foam layers resulted in more depression depth, indicating a more "cushion-like" feel. For example, the 5.5 mm sample (Example 3) rated 4.83 whereas the 3.0 mm sample (Example 1) rated 1.29.

The high density foam (Example 4) resulted in a stiffer feel. For example, the low density foam rated 2.75 whereas the high density foam rated 4.54. Fullness/volume correlated with foam thickness and density, but the non-woven also had a small influence.

Samples described above were compared to commercially available articles. The commercially available wipes were laundered to remove surfactants and other agents, and then air dried. The samples were then cut into 8 cm diameter circles when possible. Samples not large enough for cutting were evaluated in the size received. Composite samples were also cut into 8 cm diameter circles. The commercially available samples were also assessed, with the results given in Table 3.

**Table 3.**

| Sample | Sample Description | Sample Thickness (mm) | Hand Friction | Depression Depth | Fullness/ Volume | Stiffness |
|--------|-------------------|----------------------|---------------|------------------|------------------|-----------|
| Comparative 1 | NEUTROGENA® | 3.4 | 4.25 | 3.92 | 4.04 | 3.58 |
| Comparative 2 | OKAY® | 0.9 | 3.58 | 2.92 | 3.08 | 3.58 |
| Comparative 3 | PONDS® | 0.6 | 2.17 | 2.25 | 1.79 | 1.46 |
| Comparative 4 | DOVE® | 2.8 | 2.54 | 4.29 | 4.71 | 4.83 |
| Comparative 5 | BIROE'™ | 0.5 | 2.46 | 1.63 | 1.34 | 1.54 |

The commercially available samples showed a wide variation in results. The Competitive Samples 1 and 4 had higher values for depression depth, fullness/volume, and stiffness and therefore were compared directly to the samples evaluated above (Table 2). Comparative Sample 3 was also included as this represented a more typical structure available commercially. The results from this comparison are shown in Table 4.

**Table 4.**

| Sample | Sample Description | Thickness (mm) | Hand Friction | Depression Depth | Fullness/ Volume | Stiffness |
|---|---|---|---|---|---|---|
| Example 4 | Elastic nonwoven, High Density PFF | 3.5 | 5.33 | 4.88 | 4.83 | 3.92 |
| Example 3 | Elastic nonwoven, Low Density PFF | 4.3 | 5.29 | 5.79 | 5.54 | 2.88 |
| Example 7 | Soft nonwoven, Low Density PFF | 1.3 | 2.29 | 4.13 | 3.08 | 2.54 |
| Comparative 1 | NEUTROGENA® | 3.4 | 3.25 | 2.25 | 2.92 | 4.92 |
| Comparative 3 | POUNDS® | 0.6 | 2.75 | 1.17 | 1.00 | 1.42 |
| Comparative 4 | DOVE® | 2.8 | 2.25 | 2.79 | 3.63 | 5.33 |

An additional study was also conducted to include evaluation of a a lower basis weight elastic non-woven on the polyolefin foam (Example 5). In this study the characterization of the "warmth" property was included in the evaluation of the samples, with the results shown in Table 5.

**Table 5.**

| Sample | Sample Description | Sample Thickness (mm) | Hand Friction | Depression Depth | Thermal | Fullness/ Volume | Stiffness |
|---|---|---|---|---|---|---|---|
| Example 3 | Elastic nonwoven (50gms), Low Density, 5.5mm | 4.3 | 3.76 | 4.33 | 2.38 | 5.29 | 3.57 |
| Example 5 | Elastic nonwoven (25gms), Low Density, 5.5mm | 3.5 | 3.62 | 4.67 | 1.76 | 4.67 | 3.14 |
| Comparative 1 | NEUTROGENA® | 3.4 | 2.81 | 2.19 | 4.14 | 2.43 | 4.95 |
| Comparative 3 | PONDS® | 0.6 | 2.71 | 1.14 | 5.10 | 1.24 | 1.38 |
| Comparative 4 | DOVE® | 2.8 | 2.10 | 2.67 | 4.86 | 2.90 | 5.81 |

The laminated composite structures (Examples 3, 4, 5, and 7) and the resulted in higher rankings for depression depth and fullness/volume relative to the commercial samples. And, in spite of the increased volume and loft of these samples, they scored better on the stiffness ranking than the commercial competitive samples. Increasing stiffness is perceived to be a negative attribute for softness. Thus, the data demonstrates enhanced volume/loft without increasing stiffness, resulting in an overall softer feel for the composite structures.

A study was also performed to evaluate the hand feel properties of the samples with the inclusion of a liquid surfactant system. The results are shown in Table 6. All of these samples had a standard facial cleaning formulation incorporated into the wipe. The competitive samples were laundered and air dried to remove any existing cleaning agent. The cleaning formulation was incorporated into all of the samples to the same loading on a volume basis of 0.15 grams cleaning agent per 1 cm$^3$ substrate. This was done to minimize hand feel variation due to cleaning agent loading. **Table 6.**

| Sample | Sample Description | Sample Thickness (mm) | Hand Friction | Depression Depth | Thermal | Fullness/ Volume | Stiffness |
|---|---|---|---|---|---|---|---|
| Comparative 1 | NEUTROGENA® | 3.4 | 5.00 | 2.14 | 5.36 | 2.77 | 5.27 |

(continued)

| Sample | Sample Description | Sample Thickness (mm) | Hand Friction | Depression Depth | Thermal | Fullness/ Volume | Stiffness |
|---|---|---|---|---|---|---|---|
| Comparative 3 | PONDS® | 0.6 | 4.36 | 1.09 | 5.32 | 1.18 | 1.73 |
| Comparative 4 | DOVE® | 2.8 | 3.50 | 3.27 | 5.68 | 4.14 | 6.14 |
| Examples 8 | Elastic non-woven (50gms), Low Density | 4.3 | 4.68 | 5.36 | 1.86 | 5.59 | 3.77 |
| Example 9 | Elastic non-woven (25gms), Low Density | 3.5 | 3.32 | 5.50 | 2.05 | 6.00 | 3.77 |
| Example 10 | Sort non-woven, Low Density | 1.3 | 1.73 | 3.95 | 2.55 | 3.50 | 3.45 |

The inclusion of a cleaning formulation reduced the degree of perceived difference in some of the attributes evaluated, such as with the hand friction attribute. However, the same trends are evident between the dry and wet samples, specifically the Samples measured higher in the loft characteristics while maintaining a degree of flexibility than the Competitive Samples.

**Physical Property Testing**

Physical property tests are included to describe the physical attributes of the inventive sample in comparison to Comparative Samples; included are bending stiffness; fuzz level, hand, compression recovery, and tensile tear strength. Specifically the bending stiffness is a measure of the products flexibility, fuzz level is a measure of the products ability to resist wear or not to generate lint, hand is the cumulative measure of the products surface friction, compressibility, and flexibility, compression recovery is a measure of the products ability to retain loft/thickness after compression, and tensile tear strength is a measure of the strength of the product to withstand use and removal from packaging. It is desirable within the application to have a low bending stiffness; low fuzz generation, low hand on a sample volume and weight basis, high compression recovery, and good tensile tear strength.

A number of physical/mechanical property tests were performed to provide additional comparative data between the Examples and the Comparative Samples. Literature studies have shown correlations between physical properties such as tensile strength, coefficient of friction, drape, and crush measurements to correlate with the perception of softness. In addition, a rub test was performed to determine if the sample surface pills when rubbed. While this property is not associated with a soft feel, it is a negative attribute.

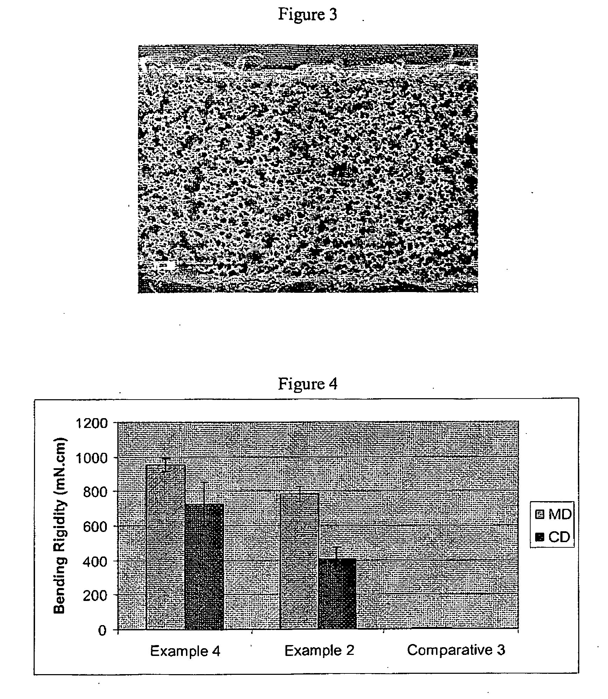

The bending rigidity test is a measure of stiffness, as it measures how long of a sample strip can hang over an edge before the strip bends. Unfortunately, several of the commercially available Competitive Samples were not of sufficient size for the bending rigidity test. Comparative Sample 3 (PONDS@) is a more simple non-woven design that is very flexible, but has low volume/fullness. Results of the bending rigidity test are shown in Figure 4. Data for the samples made from the composites of Examples 2 and 4 show that the concept with low density foam is less stiff than that with high density foam, which is in agreement with the sensory panel results given above. There is also a difference in the stiffness in the machine direction (MD) and cross direction (CD).

The rub test gives an indication of whether or not the surface of an article will pill when rubbed. Again, adequate sample size was not possible for many of the most relevant commercial samples. The rub test is performed by rubbing sandpaper of a defined grit across the surface of the sample with a controlled force. The sample is weighed before and after the test and weight loss is measured to determine the amount of fuzz formed and removed from the surface by the sandpaper.

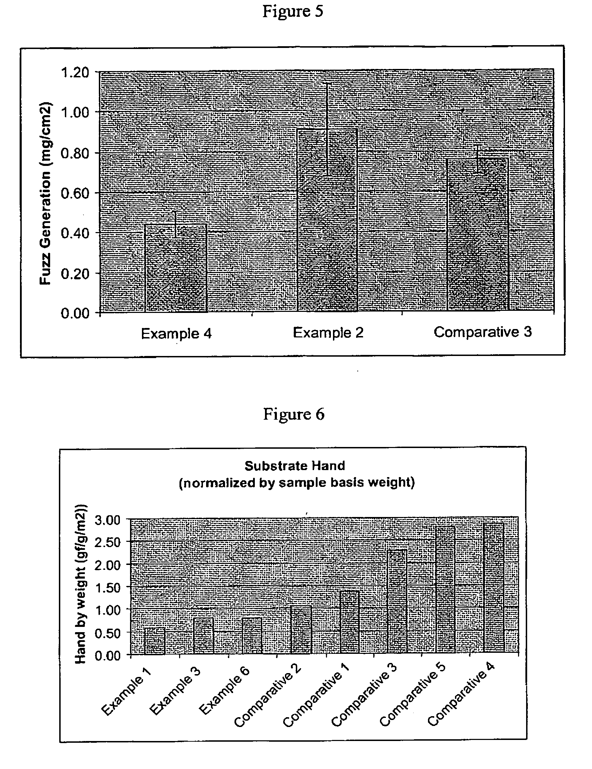

The results of the fuzz resistance test are shown in Figure 5, and indicate an improvement in the resistance of the surface to pilling with the use of an elastic non-woven substrate on the high density foam (Example 4). The Competitive Sample evaluated is a spun-laced PET. The spun-lacing process relies on mechanically intertwining the fibers to form the non-woven sheet, and no chemical or melting binders are utilized to tie fibers together. As such, these fibers are more susceptible to abrasion and to being removed from the matrix, causing a higher fuzz level measurement. The elastic non-woven utilized as described within U.S. Patent No. 6,994,763 employs melted tie points to bind the fibers of the non-woven. These bind points reduce the release of fibers during the rub test, generating a lower fuzz level, which for this type non-woven is typically 0.35 to 0.4 mg/cm$^2$. As shown, the fuzz level of this non-woven is only slightly increased to 0.44 mg/cm$^2$ when included as a laminate within the high density foam sample (Example 4). The fuzz level increases more significantly when the elastic non-woven is used in combination with the low density foam, having a fuzz level of 0.91 mg/cm$^2$. This indicates that the rigidity and/or density of the underlying material has an affect on the fuzz level of the surface substrate and that higher density foam results in a better fuzz performance, more closely approximating the performance of the base non-woven.

This data shows that the improved rub performance of the elastic non-woven compared to the spun-laced non-woven may be maintained while increasing the loft or thickness as measured in the hand panel above of the overall structure. Maintenance of this improvement is dependent on the density of the underlying material.

The measurement of "hand" is considered to be the combination of resistance due to the surface friction, flexibility, and compressibility of a fabric material. This measurement of hand has also been used to describe softness of non-woven articles (such as in U.S. Patent Nos. 6,241,780 and 6,779,718 and WO2006065563A1). The hand of a specimen is the sum of the force required to move the specimen through a slot of a fixed width in each of the four orientations as described by the instrument manufacturer. The total hand of a sample is based on an average of three specimens of the sample. This resultant total hand, in gram-force, is what is typically reported, however this assumes a level of uniformity between sample weights per unit area and volume in the comparison. Increases in total sample volume compared to a constant fixed slot width will have an effect on the total hand reported for a sample. Additionally, changes between sample weights per unit area will affect the total hand reported for a sample. To account for variation in sample volume and/or sample weight per unit area a better measure for comparison is the total hand reported for the sample on a normalized basis to these factors, volume and weight per unit area. It is desirable to have lower hand per unit volume and per unit weight per area.

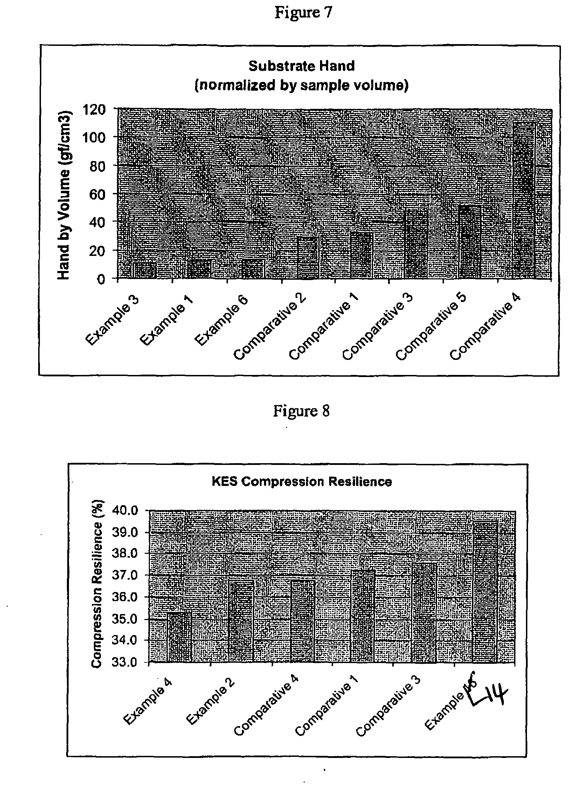

Figures 6 and 7 show the measured hand per cubic centimeter of sample and measured hand per basis weight (grams per square meter, gsm), respectively. These data show that the Examples provide lower hand values per sample basis weight and .per sample volume than the Comparative Samples. This softness/flexibility per volume/fullness is also seen in the hand panel data.

The KES compression resilience provides a measure of a materials ability to return to its original loft after compression. The higher the compression resilience the better the material can maintain loft and the perception of softness during use. Results from KES compression resilience are shown in Figure 8. The data indicates that the Examples provide similar to improved performance when compared to Comparative Samples. This improved compression resilience is achieved with higher loft, as seen in the hand panel results.

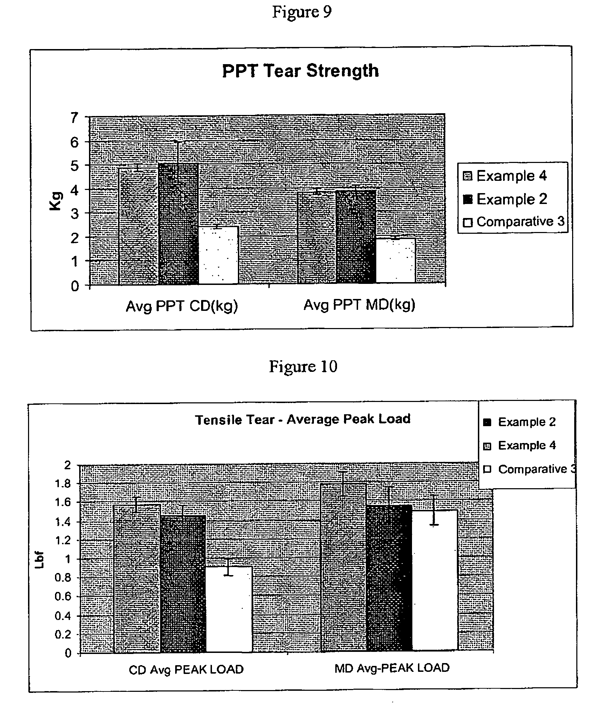

The PPT tear test provides a measure of the tear strength of the non-woven system; measured as total load, average peak load, and the average load. Results of the PPT tear test are shown in Figures 9-11. The data indicates that the Examples provide a greater overall PPT tear strength then that of the Comparative samples evaluated. A greater overall PPT tear strength is indicative of an advantage of durability within use when compared to the competitive samples. This durability advantage is achieved with greater loft, as seen in the hand panel results.

Surface properties (coefficient of friction) and surface contour (roughness) values of several Examples and Comparative samples are determined using a KES-FB4 surface tester, where a tension load of 20 gf/cm is applied to the sample. The coefficient of friction is given in MIU, a measure of the coefficient of friction on a scale of 0 to 1, where a higher MIU value corresponds to a greater friction or resistance and drag. Regarding KES surface roughness, measured in microns, a higher value corresponds to a geometrically rougher surface.

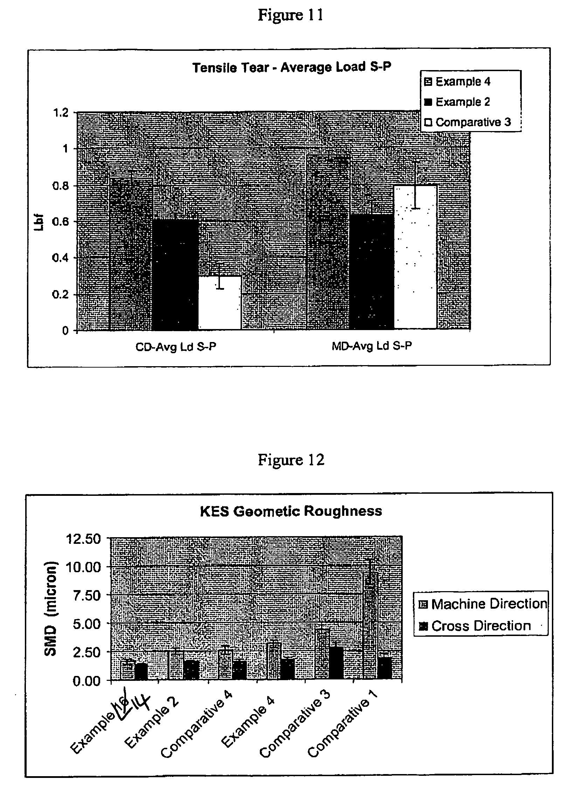

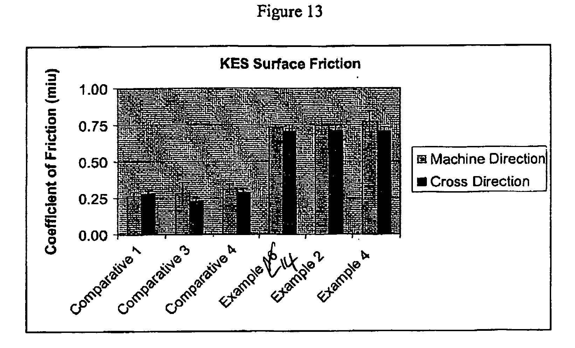

Results of the surface property tests are given in Figures 12 and 13. Figure 12 is a graphical representation of the results for Kawabata Evaluation System measurements for geometric roughness for embodiments of the composite structures disclosed herein as compared to commercially available comparative samples. Figure 13 is a graphical representation of the results for Kawabata Evaluation System measurements for coefficient of friction for embodiments of the composite structures disclosed herein as compared to commercially available comparative samples. The data indicate that the Examples are comparable to lower in surface roughness while having a greater coefficient of friction.

Advantageously, embodiments disclosed herein may provide for composite structures that have one or more substrate layers, including foams and/or non-wovens. The composite structures may advantageously allow for the softness, loft and functionality of each component to be realized in a singular or composite structure.

Advantages of embodiments disclosed herein may include increased softness, increased loft, increased toughness and the balance of these conflicting requirements. Additionally, these advantages may be met along with a greater capability to contain and deliver and active component such as a cleaning agent. Additional benefits may include the retention and reduced separation of cleaning agent during shipping and storage within the container, increase in the frothing of the surfactant in a wet system, and controlled delivery of actives.

Embodiments of the composite structures disclosed herein, including "dry" samples, may advantageously exhibit high depression depth and high fullness/volume, and low stiffness compared to higher volume comparative samples. The data for wet and dry samples indicated that embodiments of the composite structures disclosed herein may have more desirable softness attributes then comparative samples. Additionally, embodiments disclosed herein may advantageously combine a high tensile tear strength and low fuzz generation.

While the invention has been described with respect to a limited number of embodiments, those skilled in the art, having benefit of this disclosure, will appreciate that other embodiments can be devised which do not depart from the scope of the invention as disclosed herein. Accordingly, the scope of the invention should be limited only by the attached claims.

**Claims**

1.  A composite structure comprising:

    at least one substrate layer;
    at least one layer comprising an open-cell polyolefin foam having a density in the range of from 0.03 to 0.07 g/cc disposed on the substrate layer;
    wherein the substrate layer comprises a polyurethane or polyolefin non-woven having a basis weight of 15 to 250 grams per square meter; and
    wherein the composite structure has a basis weight of 15 to 500 grams per square meter.

2.  The composite structure of claim 1, wherein the substrate further comprises a pulp.

3.  The composite structure of claim 1, wherein the composite structure has a total hand per unit volume of less than 25 gf/cm$^3$, a total hand per unit weight per unit area of less than 1 gf/g/m$^2$, a Rub Test fuzz level of 0.7 mg/cm$^2$ or less, and a Kawabata Evaluation system compression resilience of greater than 35%.

4.  The composite structure of claim 1, wherein at least one of the at least one substrate layers and the at least one open-cell foam layers further comprise at least one of a cleaning surfactant, an active agent, and an enhancing filler.

5.  The composite structure of claim 1, wherein the substrate layer comprises at least one of a soft non-woven, an elastic non-woven, a fabric, a porous film, and a coated non-woven.

6.  The composite structure of claim 1, wherein the substrate layer comprises at least one of monocomponent fibers, bicomponent fibers, spunbond fibers.

7.  The composite structure of claim 1, wherein the substrate layer has a basis weight in the range from 25 to 60 grams per square meter, and a machine direction tensile strength of 10 N/5cm at a basis weight of 20 grams per square meter.

8.  The composite structure of claim 5, wherein the at least one foam layer has a thickness from 1 mm to 6 mm.

9.  The composite structure of claim 5, wherein adhesion of the at least one foam layer to the first and second substrate layers is 0.0175 N/mm (0.1 1bf/in) or greater.

10. The composite structure of any one of the preceding claims, which is suitable for use in the manufacture of a cleansing wipe.

11. Use of a composite structure of any one of claims 1 to 9, for the manufacture of a cleansing wipe.

12. The composite structure of claim 1 wherein the composite structure has a flexural rigidity of less than 100mN.cm.

13. The composite structure of claim 1 wherein the composite structure has a Kawabata Evaluation System coefficient of friction within the range of 0.1 to 0.9 MIU.

**Patentansprüche**

1.  Eine Verbundstruktur, die Folgendes beinhaltet:

    mindestens eine Substratschicht;
    mindestens eine Schicht, beinhaltend einen offenzelligen Polyolefinschaum mit einer Dichte in dem Bereich von 0,03 bis 0,07 g/cm$^3$, angeordnet auf der Substratschicht;
wobei die Substratschicht einen Polyurethan- oder Polyolefin-Vliesstoff mit einem Basisgewicht von 15 bis 250 Gramm pro Quadratmeter beinhaltet; und
wobei die Verbundstruktur ein Basisgewicht von 15 bis 500 Gramm pro Quadratmeter aufweist.

2. Verbundstruktur gemäß Anspruch 1, wobei das Substrat ferner einen Faserbrei beinhaltet.

3. Verbundstruktur gemäß Anspruch 1, wobei die Verbundstruktur eine gesamte Hand je Einheitsvolumen von weniger als 25 p/cm$^3$, eine gesamte Hand je Einheitsgewicht je Einheitsfläche von weniger als 1 p/g/m$^2$, einen Reibtest-Fusselgrad von 0,7 mg/cm$^2$ oder weniger und eine Kawabata-Bewertungssystem-Komprimierungsnachgiebigkeit von mehr als 35 % aufweist.

4. Verbundstruktur gemäß Anspruch 1, wobei mindestens eine der mindestens einen Substratschichten und der mindestens einen offenzelligen Schaumschichten ferner mindestens ein Reinigungstensid, einen Wirkstoff und/oder einen verbessernden Füllstoff beinhaltet.

5. Verbundstruktur gemäß Anspruch 1, wobei die Substratschicht mindestens einen weichen Vliesstoff, einen elastischen Vliesstoff, einen Stoff, eine poröse Folie und/oder einen beschichteten Vliesstoff beinhaltet.

6. Verbundstruktur gemäß Anspruch 1, wobei die Substratschicht mindestens Einkomponentenfasern, Zweikomponentenfasern und/oder Spinnfasern beinhaltet.

7. Verbundstruktur gemäß Anspruch 1, wobei die Substratschicht ein Basisgewicht in dem Bereich von 25 bis 60 Gramm pro Quadratmeter und eine Maschinenrichtungs-Reißfestigkeit von 10 N/5 cm bei einem Basisgewicht von 20 Gramm pro Quadratmeter aufweist.

8. Verbundstruktur gemäß Anspruch 5, wobei die mindestens eine Schaumschicht eine Dicke von 1 mm bis 6 mm aufweist.

9. Verbundstruktur gemäß Anspruch 5, wobei die Haftfähigkeit der mindestens einen Schaumschicht an der ersten und zweiten Substratschicht 0,0175 N/mm (0,1 lbf/in) oder mehr beträgt.

10. Verbundstruktur gemäß einem der vorhergehenden Ansprüche, die zur Verwendung bei der Herstellung eines Reinigungswischtuchs geeignet ist.

11. Eine Verwendung einer Verbundstruktur gemäß einem der Ansprüche 1 bis 9 zur Herstellung eines Reinigungswischtuchs.

12. Verbundstruktur gemäß Anspruch 1, wobei die Verbundstruktur eine Biegesteifigkeit von weniger als 100 mN.cm aufweist.

13. Verbundstruktur gemäß Anspruch 1, wobei die Verbundstruktur einen Kawabata-Bewertungssystem-Reibungskoeffizienten in dem Bereich von 0,1 bis 0,9 MIU aufweist.

**Revendications**

1. Une structure composite comprenant :

au moins une couche de substrat ;
au moins une couche comprenant une mousse de polyoléfine à cellules ouvertes ayant une masse volumique comprise dans la gamme allant de 0,03 à 0,07 g/cm$^3$ disposée sur la couche de substrat ;
dans laquelle la couche de substrat comprend un non-tissé en polyuréthane ou polyoléfine ayant une masse surfacique allant de 15 à 250 grammes par mètre carré ; et
dans laquelle la structure composite a une masse surfacique allant de 15 à 500 grammes par mètre carré.

2. La structure composite de la revendication 1, dans laquelle le substrat comprend en outre une pâte.

**3.** La structure composite de la revendication 1, dans laquelle la structure composite a une main globale par unité de volume inférieure à 25 gf/cm$^3$, une main globale par unité de poids par unité de surface inférieure à 1 gf/g/m$^2$, un niveau de peluchage selon le test de frottement de 0,7 mg/cm$^2$ ou moins, et une résilience à la compression selon le système d'évaluation de Kawabata supérieure à 35 %.

**4.** La structure composite de la revendication 1, dans laquelle au moins une couche parmi les au moins une couche de substrat et les au moins une couche de mousse à cellules ouvertes comprend en outre au moins soit un tensioactif nettoyant, soit un agent actif, soit une charge exaltatrice.

**5.** La structure composite de la revendication 1, dans laquelle la couche de substrat comprend au moins soit un non-tissé doux, soit un non-tissé élastique, soit un tissu, soit un film poreux, soit un non-tissé enduit.

**6.** La structure composite de la revendication 1, dans laquelle la couche de substrat comprend au moins soit des fibres à un composant, soit des fibres à deux composants, soit des fibres obtenues par filage direct.

**7.** La structure composite de la revendication 1, dans laquelle la couche de substrat a une masse surfacique comprise dans la gamme allant de 25 à 60 grammes par mètre carré, et une résistance à la traction dans le sens machine de 10 N/5 cm à une masse surfacique de 20 grammes par mètre carré.

**8.** La structure composite de la revendication 5, dans laquelle l'au moins une couche de mousse a une épaisseur allant de 1 mm à 6 mm.

**9.** La structure composite de la revendication 5, dans laquelle l'adhérence de l'au moins une couche de mousse sur les première et deuxième couches de substrat est de 0,0175 N/mm (0,1 livre-force/pouce) ou plus.

**10.** La structure composite de l'une quelconque des revendications précédentes, laquelle est adéquate pour être utilisée dans la fabrication d'une lingette nettoyante.

**11.** Utilisation d'une structure composite de l'une quelconque des revendications 1 à 9, pour la fabrication d'une lingette nettoyante.

**12.** La structure composite de la revendication 1 dans laquelle la structure composite a une rigidité à la flexion inférieure à 100 mN.cm.

**13.** La structure composite de la revendication 1 dans laquelle la structure composite a un coefficient de friction selon le système d'évaluation de Kawabata compris dans la gamme allant de 0,1 à 0,9 MIU.

Figure 1

Figure 2

Figure 3

Figure 4

Figure 5

Figure 6

Figure 7

**Substrate Hand**
**(normalized by sample volume)**

Hand by Volume (gf/cm3)

120
100
80
60
40
20
0

Example 3, Example 1, Example 6, Comparative 2, Comparative 1, Comparative 3, Comparative 5, Comparative 4

Figure 8

**KES Compression Resilience**

Compression Resilience (%)

40.0
39.0
38.0
37.0
36.0
35.0
34.0
33.0

Example 4, Example 2, Comparative 4, Comparative 1, Comparative 3, Example

Figure 9

**PPT Tear Strength**

Kg

Avg PPT CD(kg)     Avg PPT MD(kg)

Example 4
Example 2
Comparative 3

Figure 10

**Tensile Tear - Average Peak Load**

Example 2
Example 4
Comparative 3

Lbf

CD Avg PEAK LOAD     MD Avg-PEAK LOAD

Figure 11

Tensile Tear - Average Load S-P

Figure 12

KES Geometic Roughness

Figure 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 1998003713 A
- US 6028018 A
- WO 2007100312 A
- WO 2006060520 A
- WO 20041046214 A
- WO 2005019241 A
- WO 2004078900 A
- DE 4127810 A1
- DD 152729 A1
- DE 2449787 A1
- US 818911 P
- US 3590000 A
- US 3645992 A
- US 4076698 A
- US 5272236 A
- US 5278272 A
- US 6566446 B
- US 6538070 B
- US 6448341 B
- US 6316549 B
- US 6111023 A
- US 5869575 A
- US 5844045 A
- US 5677383 A
- US 5504172 A
- WO 0001745 A
- US 4762890 A
- US 4927888 A
- US 4950541 A
- WO 2004074343 A1
- WO 2005097862 A1
- WO 2005097862A1 A
- WO 2004053223 A1
- US 20040109992 A
- US 20050192365 A
- US 4793898 A, Laamanen
- US 4594130 A, Chang
- US 3585104 A
- US 5595628 A, Gordon
- US 6837970 B
- US 6824650 B
- US 6863940 B
- US 20050192402 A
- US 20040149412 A
- US 4599392 A
- US 4988781 A
- US 5938437 A
- US 6271276 B
- US 5756659 A
- US 20010011118 A
- US 4857565 A
- US 4742095 A
- US 4879322 A
- US 3437624 A
- US 5037864 A
- US 5221710 A
- US 4237264 A
- US 4092286 A
- US 5539021 A
- US 6051681 A
- US 3758643 A
- US 3806558 A
- US 5051478 A
- US 4104210 A
- US 4130535 A
- US 4202801 A
- US 4271049 A
- US 4340684 A
- US 4250273 A
- US 4927882 A
- US 4311628 A
- US 5248729 A
- US 2004027593 W
- WO 2005021622 A
- WO 20051111282 A1
- WO 2005111291 A1
- US 5246783 A
- WO 9304486 A
- US 5008204 A
- US 6994763 B
- WO 200080341 A1
- US 3929135 A
- US 4324246 A
- EP 0951228 A
- WO 2005021638 A
- US 5339021 A
- US 5688842 A
- US 5959027 A
- US 6087440 A
- US 6241780 B
- US 6779718 B
- WO 2006065563 A1

**Non-patent literature cited in the description**

- Polyurethanes, Chemistry and Technology. **SAUNDERS ; FRISCH.** High Polymers. Interscience Publishers, vol. I, 32-4244-54
- HIGH POLYMERS. 1964, vol. II, 5-6198-199

- **K. J. SAUNDERS.** Organic Polymer Chemistry. Chapman and Hall, 1973, 323-325
- Developments in Polyurethane. Applied Science Publishers, 1978, vol. I, 1-76